# EUROPEAN PATENT SPECIFICATION

(11) **EP 3 504 339 B1**
(45) Date of publication and mention of the grant of the patent: **16.03.2022**
(21) Application number: 17758468.7
(22) Date of filing: 16.08.2017
(51) Int. Cl.: C12Q 1/6883

(54) **WARFARIN SENSITIVITY ASSAY**
WARFARINEMPFINDLICHKEITSTEST
DOSAGE DE SENSIBILITÉ À LA WARFARINE

(30) Priority: 24.08.2016 GB 201614464
(43) Date of publication of application: 03.07.2019
(73) Proprietor: QuantuMDx Group Limited, Newcastle upon Tyne NE1 2JQ (GB)
(72) Inventor: O'HALLORAN, Jonathan, Uckfield Sussex TN22 5TL (GB); OSBORNE, Stephen, Newcastle Upon Tyne Tyne&Wear NE1 2JQ (GB); AREFI, Majid, Gateshead Tyne and Wear NE8 3QY (GB); SCULLY, Philip, Newcastle Upon Tyne Tyne&Wear NE1 2JQ (GB); TYSON, John, Newcastle Upon Tyne Tyne&Wear NE1 2JQ (GB)
(74) Representative: Definition IP Limited
(86) International application number: PCT/EP2017/070767
(87) International publication number: WO 2018/036881

(56) References cited:
- CN-A- 103 627 789
- CN-A- 103 710 432
- KR-A- 20110 109 627
- SEBASTIAN THOMAS ET AL: "Integrated amplification microarray system in a lateral flow cell for warfarin genotyping from saliva", CLINICA CHIMICA ACTA, ELSEVIER BV, AMSTERDAM, NL, vol. 429, 17 December 2013 (2013-12-17), pages 198-205, XP028820838, ISSN: 0009-8981, DOI: 10.1016/J.CCA.2013.12.009
- LYON ELAINE ET AL: "Pharmacogenetic testing for warfarin sensitivity", CLINICS IN LABORATORY MEDIC, ELSEVIER, US, vol. 28, no. 4, 1 December 2008 (2008-12-01), pages 525-537, XP009153393, ISSN: 0272-2712, DOI: 10.1016/J.CLL.2008.09.001
- Harsh Jayesh Sheth: "Genetic determinants of response to aspirin and warfarin and development of silicon nanowire based genotyping", , 1 October 2015 (2015-10-01), pages 1-214, XP055414187, Newcastle University - Institute of Genetic Medicine Retrieved from the Internet: URL:https://theses.ncl.ac.uk/dspace/bitstr eam/10443/3042/1/Sheth, H 2015 Compressed.pdf [retrieved on 2017-10-10]
- HELENE PUEHRINGER ET AL: "VKORC1 -1639G>A and CYP2C9*3 are the major genetic predictors of phenprocoumon dose requirement", EUROPEAN JOURNAL OF CLINICAL PHARMACOLOGY, SPRINGER, BERLIN, DE, vol. 66, no. 6, 8 April 2010 (2010-04-08), pages 591-598, XP019840357, ISSN: 1432-1041
- COSKUN SILAN ET AL: "The prevalence of1639 G>A andgenotypes in patients that requiring anticoagulant therapy in Turkish population", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 39, no. 12, 14 October 2012 (2012-10-14), pages 11017-11022, XP035133046, ISSN: 1573-4978, DOI: 10.1007/S11033-012-2004-2
- HUANG TAO-SHENG ET AL: "DNA sensors to assess the effect ofVKORC1andCYP2C9gene polymorphisms on warfarin dose requirement in Chinese patients with atrial fibrillation", AUSTRALASIAN PHYSICAL AND ENGINEERING SCIENCES IN MEDICINE, MEDICINE, MELBOURNE, AU, vol. 40, no. 1, 12 January 2017 (2017-01-12), pages 249-258, XP036198094, ISSN: 0158-9938, DOI: 10.1007/S13246-016-0519-X [retrieved on 2017-01-12]

### SEQUENCE LISTING

<110> QuantuMDx Group Limited
<120> Warfarin Sensitivity Assay
<130> P000057.WO
<150> GB1614464.4
   <151> 2016-08-24
<160> 15
<170> PatentIn version 3.5
<210> 1
   <211> 21
   <212> DNA
   <213> Artificial Sequence
<220>
   <223> Primer
<400> 1
   ggcgtttctc cctcatgacg c 21
<210> 2
   <211> 22
   <212> DNA
   <213> Artificial Sequence
<220>
   <223> Primer
<400> 2
   gagtagggtc acccaccctt gg 22
<210> 3
   <211> 22
   <212> DNA
   <213> Artificial Sequence
<220>
   <223> Primer
<400> 3
   gagtagggtc acccaccctt gg 22
<210> 4
   <211> 24
   <212> DNA
   <213> Artificial Sequence
<220>
   <223> Primer
<400> 4
   gaatttaatg tcacaggtca ctgc 24
<210> 5
   <211> 21
   <212> DNA
   <213> Artificial Sequence
<220>
   <223> Primer
<400> 5
   ggattatagg cgtgagccac c 21
<210> 6
   <211> 24
   <212> DNA
   <213> Artificial Sequence
<220>
   <223> Primer
<400> 6
   ggtaggtgca acagtaaggg atcc 24
<210> 7
   <211> 19
   <212> DNA
   <213> Artificial Sequence
<220>
   <223> Probe
<400> 7
   gaacacggtc ctcaatgct 19
<210> 8
   <211> 19
   <212> DNA
   <213> Artificial Sequence
<220>
   <223> Probe
<400> 8
   gaacacagtc ctcaatgct 19
<210> 9
   <211> 23
   <212> DNA
   <213> Artificial Sequence
<220>
   <223> Probe
<400> 9
   acgaggtcca gagatacatt gac 23
<210> 10
   <211> 22
   <212> DNA
   <213> Artificial Sequence
<220>
   <223> Primer
<400> 10
   cgaggtccag agataccttg ac 22
<210> 11
   <211> 16
   <212> DNA
   <213> Artificial Sequence
<220>
   <223> Probe
<400> 11
   caaccattgg ccgggt 16
<210> 12
   <211> 16
   <212> DNA
   <213> Artificial Sequence
<220>
   <223> Probe
<400> 12
   caaccattgg ccaggt 16
<210> 13
   <211> 29
   <212> DNA
   <213> Artificial Sequence
<220>
   <223> Primer
<400> 13
   ctgaatttaa tgtcacaggt cactgcatg 29
<210> 14
   <211> 4158
   <212> DNA
   <213> Homo sapiens
<400> 14
   gcgccgacta caactcccat catgcctggc agccgctggg gccgcgattc cgcacgtccc      60
   ttacccgctt cactagtccc ggcattcttc gctgttttcc taactcgccc gcttgactag     120
   cgccctggaa cagccatttg ggtcgtggag tgcgagcacg gccggccaat cgccgagtca     180
   gagggccagg aggggcgcgg ccattcgccg cccggcccct gctccgtggc tggttttctc     240
   cgcgggcgcc tcgggcggaa cctggagata atgggcagca cctgggggag ccctggctgg     300
   gtgcggctcg ctctttgcct gacgggctta gtgctctcgc tctacgcgct gcacgtgaag     360
   gcggcgcgcg cccgggaccg ggattaccgc gcgctctgcg acgtgggcac cgccatcagc 420
   tgttcgcgcg tcttctcctc caggtgtgca cgggagtggg aggcgtgggg cctcggagca 480
   gggcggccag gatgccagat gattattctg gagtctggga tcggtgtgcc cggggaacgg 540
   acacggggct ggactgctcg cggggtcgtt gcacaggggc tgagctaccc agcgatactg 600
   gtgttcgaaa taagagtgcg aggcaaggga ccagacagtg ctggggactg ggattattcc 660
   ggggactcgc acgtgaattg gatgccaagg aataacggtg accaggaaag gcggggaggc 720
   aggatggcgg tagagattga cgatggtctc aaggacggcg cgcaggtgaa ggggggtgtt 780
   ggcgatggct gcgcccagga acaaggtggc ccggtctggc tgtgcgtgat ggccaggcgt 840
   tagcataatg acggaataca gaggaggcga gtgagtggcc agggagctgg agattctggg 900
   gtccagggca aagataatct gcccccgact cccagtctct gatgcaaaac cgagtgaacc 960
   gttataccag ccttgccatt ttaagaatta cttaagggcc gggcgcggtg gcccactcct 1020
   gtaatcccag cactttggga ggccgaggcg gatggatcac ttgaagtcag gagttgacca 1080
   gcctggccaa catggtgaaa gcctgtctct accaaaaata gaaaaattaa tcgggcgcta 1140
   tggcgggtgc cttaatccca gctactcggg ggggctaagg caggagaatc gcttgaaccc 1200
   gggaggcgga ggtttcagtg agccgagatc gcgccactgc actccagcct gggccagagt 1260
   gagactccgt ctcaaaaaaa aaaaaaaaaa aaaaaaaaaa gagacttact taaggtctaa 1320
   gatgaaaagc agggcctacg gagtagccac gtccgggcct ggtctgggga gaggggagga 1380
   tagggtcagt gacatggaat cctgacgtgg ccaaaggtgc ccggtgccag gagatcatcg 1440
   acccttggac taggatggga ggtcggggaa cagaggatag cccaggtggc ttcttggaaa 1500
   tcacctttct cgggcagggt ccaaggcact gggttgacag tcctaacctg gttccacccc 1560
   accccacccc tctgccaggt ggggcagggg tttcgggctg gtggagcatg tgctgggaca 1620
   ggacagcatc ctcaatcaat ccaacagcat attcggttgc atcttctaca cactacagct 1680
   attgttaggt gagtggctcc gccccctccc tgcccgcccc gccccgcccc tcatccccct 1740
   tggtcagctc agccccactc catgcaatct tggtgatcca cacagctgac agccagctag 1800
   ctgctcatca cggagcgtcc tgcgggtggg gatgtgggga ggtaactaac aggagtcttt 1860
   taattggttt aagtactgtt agaggctgaa gggcccttaa agacatccta ggtccccagg 1920
   ttttttgttt gttgttgttt tgagacaggg tctggctctg ttgcccaaag tgaggtctag 1980
   gatgccctta gtgtgcactg gcgtgatctc agttcatggc aacctctgcc tccctgccca 2040
   agggatcctc ccaccttagc ctcccaagca gctggaatca caggcgtgca ccactatgcc 2100
   cagctaattt ttgtttttgt ttttttttgg tagagatggt gtctcgccat gttgcccagg 2160
   ctggtctcaa gcaatctgtc tgcctcagcc tcccaaagtg ctggggggat tacaggcgtg 2220
   agctaccatg ccccaccaac accccagttt tgtggaaaag atgccgaaat tcctttttaa 2280
   ggagaagctg agcatgagct atcttttgtc tcatttagtg ctcagcagga aaatttgtat 2340
   ctagtcccat aagaacagag agaggaacca agggagtgga agacgatggc gccccaggcc 2400
   ttgctgatgc catatgccgg agatgagact atccattacc acccttccca gcaggctccc 2460
   acgctccctt tgagtcaccc ttcccagctc cagagaaggc atcactgagg gaggcccagc 2520
   accatggtcc tggctgacac atggttcaga cttggccgat ttatttaaga aattttattg 2580
   ctcagaactt tccctccctg ggcaatggca agagcttcag agaccagtcc cttggagggg 2640
   acctgttgaa gccttctttt tttttttttt taagaaataa tcttgctctg ttgcccaggc 2700
   tggagtgcag tggcacaatc atagctcact gtaacctggc tcaagcgatc ctcctgagta 2760
   gctaggacta taggcatgtc actgcaccca gctaattttt tttttttttt tttttttttt 2820
   tttgcgacat agtctcgctc tgtcaccagg ctggagtgca gtggcacgat cttggctcac 2880
   tgcaacctct gcctcccggg ttcaagcaat tttcctgcct cagcctcctg agtagctggg 2940
   actacaggcg cgtgtcacca cgcccagcta atttttgtat ttttagtgga gacagggttt 3000
   caccatgttg gctaggatgg tctcaatctc ttgacctggt gatccatccg ccttggcctc 3060
   ccaaagtgct aggattacag gcgtgagtca acctcaccgg gcattttttt tttgagacga 3120
   agtcttgctc ttgctgccca agctggaatg tggtggcatg atctcggctc actgcaacct 3180
   ccacctccta ggttcaagcg attctccacc ttagcctccc cagcagctgg gattacaggt 3240
   gcccatcaac acacccggct aatttttgta tttttattag agatggggtt ttgccatgtt 3300
   ggccaggctg ctctcgaact cctaacctca ggtgatccac ccccattggc ctcccaaaat 3360
   actgggatta caggcatgag ccaccgtgcc cagctgaatt tctaaatttt tgatagagat 3420
   cgggtctttc tatgttgccc aagctggtct tgaactccta gcctaaagca gtcttcccac 3480
   ctcggcctcc cagagtgttt ggaatacgtg cgtaagccac cacatctgcc ctggagcctc 3540
   ttgttttaga gacccttccc agcagctcct ggcatctagg tagtgcagtg acatcatgga 3600
   gtgttcggga ggtggccagt gcctgaagcc cacaccggac cctcttctgc cttgcaggtt 3660
   gcctgcggac acgctgggcc tctgtcctga tgctgctgag ctccctggtg tctctcgctg 3720
   gttctgtcta cctggcctgg atcctgttct tcgtgctcta tgatttctgc attgtttgta 3780
   tcaccaccta tgctatcaac gtgagcctga tgtggctcag tttccggaag gtccaagaac 3840
   cccagggcaa ggctaagagg cactgagccc tcaacccaag ccaggctgac ctcatctgct 3900
   ttgctttggc atgtgagcct tgcctaaggg ggcatatctg ggtccctaga aggccctaga 3960
   tgtggggctt ctagattacc ccctcctcct gccatacccg cacatgacaa tggaccaaat 4020
   gtgccacacg ctcgctcttt tttacaccca gtgcctctga ctctgtcccc atgggctggt 4080
   ctccaaagct ctttccattg cccagggagg gaaggttctg agcaataaag tttcttagat 4140
   caatcagcca agtctgaa 4158
<210> 15
   <211> 57734
   <212> **DNA**
   <213> Homo sapiens
<400> 15
   aaacatagta tcatttatat tagcaccaaa aatatgaaat ccctaggtgg aagtctgtca      60
   aaatacatat atcacttgaa gaaaactaca aaactctgat gaaagaaaac aaagatataa     120
   ataaatacac aaatatttca tgttcatgga tatgaagacc gttgttagtc tttttgattt     180
   gatctataga tcaacacaat aagaatcaaa ctccaggaaa gttttattat ttttattatt     240
   ttttatcttt agatgctaac aatctgattg taaagtttat gtggagaggc caaaaaaccc     300
   agaatagcca acgttaagga gaacaaagtc aaatgactga ccttacccaa ttcaacttca     360
   agacttacta tgaagctaat caagacagtg tgcaattggt gaaaaaagtc aaatagatta     420
   atgcaagtga gtaaagagtc tgaagtagat catcagaaat atagtcaact gatctttgaa     480
   aaaaaaggaa agacaattca atgggggaaa aatggtcttt tcaacgaaga ctaatggagt     540
   aactggaggt tcacatacgg aaaaatgaat ctggaaatag accttacacc tttaatgaaa     600
   gttaactaaa aatatattat agatctaagc cgggtgcagt ggcttatgcc agtattccca     660
   gtactttggg aggctgaggt aggcagatca caggtcagga gatcgagacc atcctggcta     720
   acacggtgaa acccccatct ctactaaaaa tacaaaaaat tagctgggca tggtggcacg     780
   cacctgtagt cccaggttgt caagaggctg aggcaggaga atcgcttgaa ctcaggaggc     840
   agaggttgaa gtgagccgag ttgagagcca ctgcactcca gcctaggtga cagagtgaga     900
   ctcagtctca ggaaaaaaaa aaaaaaaaaa aaaaatatat atatatatat atgtgtatat     960
   atatatatat gtgtatatat atatatatgt gtatatatat atatatatat atatacgtgt    1020
   gtgtatatat atatatacat atatatatta gatccaaata taaaatgcaa aactatgaat    1080
   ctctgatagt ataagatagg agaaaataaa gatgaccttg atcagcaatg gtttttaaaa    1140
   tactacaatg aaggtataat ccatgaaata aagaatgact aagctggacc ttattaaaat    1200
   ttaaacttct actctgcaaa agacactatc aagggaatga gatgaaaaga cagagactgg    1260
   gagaaatact ttcaaaagat atatctgata aacgacttct ctccaaaaat ttttaaaact    1320
   ccacaattag aaatcagaca ccttgattta aaaaatgggc aacagatctg aacagacacc    1380
   tcacccaaga agaaatacag atggcaaata aatatatgaa aaatgctcat cattaaacgt    1440
   cactacggac ctgcaaacta aaaccccaat gagatacaac tacacactta tacaatggct    1500
   aaaatccaaa acactgataa aaccaaatgt tgacaaggat gtgcagcaat gagaactaag    1560
   tcattgctgg tggaaatgca aaatgataaa gacacttcag gagacagatg ggcagtgcct    1620
   aaaaaaacta aaatatgttt accacattat ctggtagttg tgctctttgg tatttgccaa 1680
   ataagttgaa aatacatttc cacacaaaaa tctgcacatg gatatttata gcagctttat 1740
   tcatcattgt caaaaattag aaatcaccaa gatgttcttc aatagataaa tgaatagaca 1800
   acgtgataaa cctatacaat gaatgttatt tagtgttaac aaggaatgaa ctatcaagcc 1860
   atgaaaagac atgcagaaac tttatacata tgagtaagtt aaatcttaaa aggctacata 1920
   ctgtatgatt ccaaccgtat tacattttgg aaaaggcaaa actatgccta cactgcaaag 1980
   attatttgtt gctagggctt gcaaggaagg gagagagaac acgtaaagca cagaggttct 2040
   tagggcagta aagctatttt atgtgatatt acaatggcac atgtcattat gcattagtca 2100
   aatcccatag aatgtacaac acaaagaatg aaccctacat aaactatgag ctttggttga 2160
   tgatgatgcg tcaatgttgg ttcattgatt gtaactaatg agagttcttg attgtgagag 2220
   agactgtgtg tgggtgcagg gaaagaggca tgtggtaatt atatactttc cacttagtgt 2280
   tgccagaaac cccaaactgt ttcaaaagcc tactctaatc caccattcta gtcaagctga 2340
   ggtttgtatt actcagtgac tgtggagggc ttaatgttga tactcccctg atcatttgga 2400
   ctgagatgtg aattctatga gatgggatgt gacatgtcac tgaattggga gttgaaaaac 2460
   tgggattcta agaaaagtct tggctgggcg ttgtggctca tgcctgtaat cccagcactt 2520
   tgggagacct aggtgggtgg atcacaaggt cgggagatca agatcatcct gcccaacatg 2580
   gtgaaaccct gtctgtacta aaaatacaaa aaaattagct gggcgtggtg gtgggcacct 2640
   gtagtcccag ctacttggga ggctgaggca ggagaatggc atgaacccag gagctggaaa 2700
   ttgcagtgag ccgagatcgc accactgcac tccagcctgg gtgacagagc gagactctgt 2760
   ctaaaaaaaa aaaaagaaaa gaaaaagtct tatactgagg cattgtgatt gtgatacttt 2820
   gtctcactga gtcaataatt gctcatttct taaaaaaaaa aaagcaaagt ttagagtagt 2880
   tgatctcaga tatcccttct atctacacat tatctataat tctttctttc tgtaaactga 2940
   aaggtctagg aaggagccgc agctcagcag gagagaggag gagctgagct gggaccccta 3000
   cctcctgagg aatgaaatga ttattataaa gacagcaacc gagcttattt tacccaaaat 3060
   aaggtagtat atttctgtta gagtttagag tttcatgagt cagggaccaa gttattgctt 3120
   ttctttgccc tgtataaagg cttctccaag gcctttgact tacctaagta ctaaatgtta 3180
   taaaaccaaa ctcttctgac ctctcaatct agtcaactgg ggctgtaatt attaatgaaa 3240
   ttaatgttta ttttgaaaat aatttactag actgaattac gaaatcctga atcattgtac 3300
   actatcagta aatattggtg gacccaactg aactgaatgt tttgcttgaa atgaaacctt 3360
   tgagatgcag ggcttatggg ttctagtccc agctctagca ctagcagaca gcatgttctt 3420
   ggctaagata ctgaatcttc aaggctcagc ttcctcattc cggaaatggg tcaattttat 3480
   tgtaagcaga ggtaattgag agattcaaaa gggacatgag gtgtaacaat tctctgtaaa 3540
   ttgttagaat ccctgttaaa aatgaccagt aaagctttgt gcaactgtgt cttgacataa 3600
   ctttattttt cttaataaaa gaaatggaaa taacctcact agggaattta gaacaaatat 3660
   gatgatatct ttaaagaaaa tggctttgca caagtattga cattaatgat ctagtaaagt 3720
   gtatctttct agttgtattt agatcctcaa ctcagtatgt cagctcctgt taaggtctat 3780
   acattgtggt ggttctgtgc tgtgggtcca tttagtgatt tccctacctc ccatctttta 3840
   ttgcatccac aactgtggtt ctgtccataa tttcctttgc tttctgtgca ttattacatc 3900
   atatctgaaa atgagaaacc aaaaacaata gaaagcagcc atgtctggag gtgactgggg 3960
   ggtcgagaag ccctagtttc tcaaaccctt agcaccaaat ttttccctca gttacactga 4020
   gcgtttcact tctgcagtga tggagaaggg agatccctta tttcttctca tgagcatctc 4080
   tggtgctgtt tcccttagag acaaataagg ggttctattt aatgtgaagc ctgttttatg 4140
   aacagaataa atgtggtgta tattcagaat aactaatgtt tggaagtttg ttttattttg 4200
   ctaaaattgt tctcaaggca gctctggtgt aagagataat acaccacgat gggcatcaga 4260
   agacctcagc tcaaatccca gttctgccag ctatgagctg tgtggcacca acaggtgtcc 4320
   tgttctccca gggtctccct tttcccattt gaaaaataaa aaataacaat tcctgccttc 4380
   aggaattttt tttagggggt ttaatggtaa aggtgtttat atctgctaag gtaatttact 4440
   tgatatatgt ttggttattt aagatatatg agttatgtta gctatttcat gtttaggctg 4500
   ctgtattttt agtaggctat attaaatatt tgaaaggatt tcattataaa gaacaaagtc 4560
   tcctaatctt tgatatagca ttgacatact ttttaaatat acaaggcata gaatatggcc 4620
   atttctgtta aatcatatat tcccaactgg ttattaatct aagaattcag aattttgagt 4680
   aattgctttt gcatcagatt atttacttca gtgctctcaa ttatgatggt gcattagaac 4740
   catctgggtt aacatttgtt ttttattacc aatacctagg ctccaaccaa gtacagtgaa 4800
   actggaatgt acagagtgga caatggaacg aaggagaaca agaccaaagg acattttatt 4860
   tttatctgta tcagtgggtc aaagtccttt cagaaggagc atatagtgga cctaggtgat 4920
   tggtcaattt atccatcaaa gaggcacaca ccgaattagc atggagtgtt ataaaaggct 4980
   tggagtgcaa gctcatggtt gtcttaacaa gaagagaagg cttcaatgga ttctcttgtg 5040
   gtccttgtgc tctgtctctc atgtttgctt ctcctttcac tctggagaca gagctctggg 5100
   agaggaaaac tccctcctgg ccccactcct ctcccagtga ttggaaatat cctacagata 5160
   ggtattaagg acatcagcaa atccttaacc aatgtaagta tgctccttca gtggcttgca 5220
   aaaggtaagt aaattcacct gtatttttta aataaagtgt atccctagag gtacatgtta 5280
   caagaggtaa tggtaaagta aaatactttg aaaggctttt gttgcctttt ccagtctgtc 5340
   agtgtcagaa atagtggaat gaaaccatgt attttgtgag tagagaaaga tttgggtctt 5400
   tgcatgttag attcaaaata acaagtgtca atagtttgaa aagctgtgtt ccttcttcat 5460
   ttcataacca tttgctataa tttttggctg aaggtaaatg gtaaggtatt gtgggatctg 5520
   gtcagcagcc cacaaagcaa ctgggctctc tcttttttcc caggtggatc ggcaggttga 5580
   gaaataatag acacacaaga tagtgaaagc tgggtccagg ggggtcaccg ccttctggtc 5640
   ccatggtgcc aagaatgcac tggatatacc agcatttatt attaagttta gtgagggcag 5700
   gggtaggtta gtgagggatt tagggtcatt tgattatgag gtgagatggt cacatgggga 5760
   tgaagtaatt ctttaacata acatttgtat gtagaagtac agtacatttg tatgtagaag 5820
   tacagtatac ggagataaga atttacaata tagtgtgtgc gtcagtaatt tctaacagag 5880
   ccttaaaaca gaaacacaat ctttccataa cctatgatta gcaagatatt aatcagcagt 5940
   aacaattgca acaaaagctg gttacaaaca atccatggaa ataggatgtg aagctagaca 6000
   accagttaga ccagaaattc tcagaaggga gtatgcctta accctaaaga ggcctagaag 6060
   agccatggca agatgagggc atttatagcc ctatcttatc catatggaca ggtgcccccc 6120
   atgcgtccat ttataggttc tccacaaagg tcgcattcca ttcccagagc tatgaacatc 6180
   tgcttttctg ggataggaat cttggtgatg tgaaacctct ctgactgcac gtccattcat 6240
   aggctctctg cagggggaag cacatcacgc gctgttggct cattctggca gtccagcctg 6300
   gcactgtcct tacacaatcc tgcatgcaat tttgtattta caataatcgg gagcatttca 6360
   tcttttattc cgtagcaata gtttcagggg gggtctccct acagtaagga acatgtttgt 6420
   tattagagat tttattaaat aagtcctcta ctatattagc catgtgtttt attcagaata 6480
   gccatgaaaa ttaaacttct ctgaagaatt aatttatgcc tgttgtaaag gaaataactg 6540
   tgggttcagg accagcaaaa gcagataagt ggtagaaaag aagttataga cttccatttt 6600
   tagatccaca aagtactgtt ttaattatac tatagctaca atatataaac cccaaaatga 6660
   ccttagcatt tagtaatggt aggaagaagg tcagaagatg tataagtatt agggtttttt 6720
   ctcaagtgaa agatggttta gcagtgttaa tatgatatgt gaacacccct cgttgtgtaa 6780
   taattaccat atacagagag taaagggctg caattagtat attaagtctt ataagcatga 6840
   tggtatgatt agatcaagag aatgacgctg tagttatgaa gactactcct actagattga 6900
   tggtaggggg cagggcaagg ttggcaagac tagctaggag tcatcacatg gctatcaggg 6960
   agagaagtgt ttgaaggcct tgtgttagta gtatggtttg gctgtgaact cgtttgtagt 7020
   ttgagtttgc aaggcagaat aatagggatg aggttagtct gtgggcagtt attagggaag 7080
   ttgcactggt aaaacttcag ggggtttgaa tgagaaaagc tacgataaca agtgccatgt 7140
   ggcttatgga aaagtaatga gtgattttag atcagtttgg cacagacaaa aggagcttgt 7200
   tatgattatt tctcaaaagg atagtaagaa ggaggaataa gctataaact ctgttagggg 7260
   gttaagaatt aaggtgattt gtataatcct atagccatct aattttagga gtactgctgt 7320
   gaggactatt gacctggcaa ttgcggcctc tatgtgggct tttgggagtc ctaggtggag 7380
   tccatagaga gatattttta gtataaatgc tatgatgcat gctaatcata aaaggctatt 7440
   aaaccaggaa attgttagtt cttgaagctg ggtattggtc ttaaggtaga ctcgaaagtg 7500
   ctgataaatt tctcaagcat cagtgtttga ataagcggag tttcaaattt tggtctgctg 7560
   tacattagct gtgagacact gaaaatgaat ttgtcattct ctgagctcag tttttttttt 7620
   tttttttttt ttttttgaga cagagtctta ctctgtagct caggctggag tgcagtggta 7680
   caatcttggc tcactgcaac ctccatctcc caggtcccca ttcaagaaat tctcctgcct 7740
   cagtccccca agtagctagc attacaggca tgcaccacca tgctcagcta atttttgtat 7800
   ttttagtaga gacgtggtat caccttgttg gccaggctgg tcttgaactc ctgaccttgt 7860
   gatccacctg ccttggcctc ccaaagtgtt gggattacag gcaggagcca ccacacctgg 7920
   ccgtttgttt aaaatagagt aaatagacct gctgaatatg ttgatgtgag tattaattgt 7980
   aatctgcata gcaattgtct gaccattgcc ttgaacatca caggccatct gagtggcaag 8040
   tataatcatc atcatgtttc tatttaaaat tcagaaatat ttgaagcctg tgtggctgaa 8100
   taaaagcata caaatacaat gaaaatatca tgctaaatca ggcttagcaa atggacaaaa 8160
   tagtaacttc gtttgctgtt atctctgtct actttcctag ctctcaaagg tctatggccc 8220
   tgtgttcact ctgtattttg gcctgaaacc catagtggtg ctgcatggat atgaagcagt 8280
   gaaggaagcc ctgattgatc ttggagagga gttttctgga agaggcattt tcccactggc 8340
   tgaaagagct aacagaggat ttggtaggtg tgcatgtgcc tgtttcagca tctgtcttgg 8400
   ggatggggag gatggaaaac agagacttac agagctcctc gggcagagct tggcccatcc 8460
   acatggctgc ccagtgtcag cttcctcttt cttgcctggg atctccctcc tagtttcgtt 8520
   tctcttcctg ttaggaattg ttttcagcaa tggaaagaaa tggaaggaga tccggcgttt 8580
   ctccctcatg acgctgcgga attttgggat ggggaagagg agcattgagg accgtgttca 8640
   agaggaagcc cgctgccttg tggaggagtt gagaaaaacc aagggtgggt gaccctactc 8700
   catatcactg accttactgg actactatct tctctactga cattcttgga aacatttcag 8760
   gggtggccat atctttcatt atgagtcctg gttgttagct catgtgaagc gggggtttga 8820
   agctgagagc caagggaatt tgcacatatt tgtgctgtgt gtgtacaggc atgattgtgc 8880
   gtacagtgtg ggtataaaag gttcatttaa tcccatgttc tcctgaactt tgcttttttg 8940
   ctttcaaata agaaatgatg aatatagatt ttgagttcat tttttgaaag agttaaagag 9000
   cagtgttttt cccattacct attccagaac atgtcaccag agaatacttg acaagtcaac 9060
   atggtgggaa tggccctatc atacccatat ggagcatgaa ccaaatggca tgtgctttta 9120
   tttaattgga ctgtgtttgt atggtcagcc tcactgactt ctctggggtt tcttttaggc 9180
   ccgtgcttgc cattctggcc agtaatgaca ttctacagtt tttattgctt aggcatatct 9240
   tagtgcagtt ctcatcaatt attatttctc tgtaaacaca gcattatttt aaaaatagta 9300
   ttaattattt cttgttactg tattgattta tatattttca gtaaatacat cctgtagcat 9360
   aattctgtga aatacccaaa tgtcaattta taaaatgatt tatttaacaa gattttactt 9420
   attagtaata actctgtaat ctgcattccc tatgtatgat ttggctctgt ttcagttttg 9480
   cttatctctt tccaaccata tttatgaaat tttggcttag aaatttatgt taattatttt 9540
   ttttccatgg ccaactctac tcatctatga agttttacaa tgaatctgtt tatcagcttg 9600
   gataccaaat taccttgttt ttaaattctg ttttcccaat gaagttaaag actgaaaatc 9660
   agatttatct gtgaatgaca cacacaaact aacagatttc caactgttca atgcctggcc 9720
   attcattcag agtacttttg attaaagtca ctatttaggg cctatagata tcaggggaaa 9780
   ttcagcccct gatatttcaa tgtgagtcct tttctattct ccctaagtgt tggctggtct 9840
   gagaaataaa gggaaagagt acaaaagaga gaaattttaa agctgggtgt ccaggggaga 9900
   catcacatgt cggcaggttc cgtgatgccc cctaaaccac aaaaccagca agttttcatt 9960
   agtgattttc aaaagcggaa ggaatgtgtg aatagggtgt gagtcacaga gatcacatgc 10020
   ttcacaaggt aataaaatat cacaaggcaa atggaggcag ggtgagatca caggaccgga 10080
   gtgaaattaa aattgctaat gaagtttcag gcactcattg tcattgataa catctcatca 10140
   ggagacaggg tttgagagaa gacaaccagt ctgaccaaaa tttattaggc aggaatttcc 10200
   tcttcctaat aagcctggga gcactatggg agaccaggcc ttatttcatc ccttatctac 10260
   aactgtaaaa gacagacgtt ctcaaaacag ccattttaga gaccacccct tgggaatgca 10320
   ttcttctcag ggatgttcct tgctgagaaa aagaattcag tgatatttct cctacttgct 10380
   tttgaaagaa gtgaaatatg gctctgttct gctcagccca caggcagcca gacttcaagg 10440
   ttatctcact tgttccctga acatcgctgt tatcctgttt tttttttcaa ggtgcagatt 10500
   tcatattgtt taaacaattt gtgcagttaa cacaatcatc acagggtcct gaggtgacat 10560
   tcgtcctcag cttaggaaga tgatggtatt aagagattaa agtaaagaca ggcataggaa 10620
   atcacaagag tattgattgg ggaagtggta agtgtccatg aaattttcac aatttatgtt 10680
   cagagattgc agtaaagaca ggtgtaagaa attataaaag aatgaatttg gggaactaat 10740
   aaatgtctgt gaaatcttca caatttatct tcttctgcca tggcttcagc tggtccctcc 10800
   gttcggggtc cctgacttcc tgaaacatat agatgtgaat ttgggagctc tttaactata 10860
   aagtttaata tctcaaaata ataagagcta tttatgacaa acccatagcc aatatcatat 10920
   tgaatgggca aaagctggaa gcattccctt tgaaaaccag cacaagacaa ggatgccctc 10980
   tctcaccact cttattcaac atagtattgg aagtcctggc cagagcaatc agtcaagggg 11040
   gtattcaaat gggaagagaa gaagtcaaat tgtctctgtt tgcaggtgac atgactgtat 11100
   acttagaaac cccatcatct cagccccaaa actgtttaag ctgataagca atttcagcaa 11160
   gacctcagga tacaaaatca atgtgcaaaa ataacaagca ttcctataaa ccaataatag 11220
   acaagcagag agccaaatca tgagtggact cccattcaca attgctacaa agggaataaa 11280
   atgcctactt acacaactta caagcgatgt gaaggacctc ttcaaggaga actacaaacc 11340
   acttctcaag gaaataagag gtgacacaaa tggaaaaaaa ttccgtgctc atgaatagaa 11400
   agaatcaata ctgtgaaaat agccatactg cccaaagtaa ttcatagatt caatgctata 11460
   cccgtcaaac tatcattgac tttcttcaca gaactagaaa agaataattt aaatttcata 11520
   tgaagcaaaa aaagagcctg tatagccaag acaatcctaa gcaaaaacaa agctggaggc 11580
   atcattctac ctgacttcaa acaatactac aaggctacag taaccaaaca gcatggtact 11640
   gggaaaactg gctagccata tgcagaaaac agaaactgga ccctttcctt acaccttata 11700
   caaaaattaa ctcaagatgg attaaacact taaacataaa accaaaaacc ataaaaaccc 11760
   cagaagaaaa cctaggcaat accattcagg acataggcat gggccaagag tttgtgacta 11820
   aaacacaaaa agcaatggca acaaaagcca aaattgacaa atgggatcta attaaactac 11880
   agtgcttctg cacagccaaa gaaactgcat cagagtcaac aggcaaccta cagaatggga 11940
   gaaatttttt gcaatctatc catctgacaa agggctaata tccagaatcc acaaagaact 12000
   taaatttaca agaaaaaaag aaatatcccc atcaaaaatg gacaaaggat atgaacagat 12060
   gcttctcaaa agaagacatt tatgcagcca acaaacatat ggaaaaaagc ttatcaacac 12120
   tggtcattag agaatgcaaa tcaaaactac aatgagatac catctcatgt cagttagaat 12180
   ggtgatcctt actaagtcag gaaacaacag gtgttggtga gaatgttgag aaatatgaac 12240
   cctcttacac tgttggtagg agtgtaaatt agttcaacca ttgtggaaga cagtgtggtg 12300
   attccttaag gatctagaac tagaaattcc acttgactca gcaatcccat tactgggtat 12360
   atgcccaaag gattataaac cattctacta taatgacaca cgcccatgta tgtttatgtg 12420
   gcaccgttca caatagcaaa gacttggaac caacccaaat gcccatcaat gatgtactgg 12480
   ataaagaaaa tgtggcacat atacaccatg gaatactaca cagccataaa aaaggatgag 12540
   ttcatgtcct ttgcaggaac atggatgaag ctggaaatca tcatcctcag caactaacac 12600
   aggaacagaa aaccaaactc cacatgtttg cactcataaa tgggagttga acaatgagaa 12660
   cacatggaca cagggagggg aacatcacac atgggacctg ttggggcacg gggggccagc 12720
   agggggacag cattaggaca aatacctaat gcgtgtgggg cttaaaacct agatgacagg 12780
   ttcataggtg cagcaaacca ccatggcaca tgtatacctc tgtaacaaac ctgcacgctc 12840
   tgcacatgta tcctggaact taaagtaaaa taaaagaaaa tttaaaaaat ataaagttta 12900
   tcgatcactt gattataaca acttcatttc agagatcctc caccaaacaa gaaaactttt 12960
   cctataattt aattcattta gctattaaat gatacgctgg gcctaatcaa tatcaaatta 13020
   aataggttaa atttgcagga tgattagaga tgagggcatt tagttcctaa agaagaaaat 13080
   tcattttaaa aaaggtacag aagaaaaaaa ctccacttat caatttacac tttcttgttt 13140
   gccttacctg ggatttatat ttaaaataaa agtttttggc caggcgcggt ggctcatgcc 13200
   tgtaatccca caactttggg aggccaaggc tggtggatca tctgaggttg ggagttcgag 13260
   accagcctga ccaacatgga ggaacctgtc tctactaaat atacaaaatt agctggatgt 13320
   ggtggcacat gcctgtaatc ccagctacct gggaagctca ggtggagaat cacttgaacc 13380
   taggaagcag aggttgtggt gagctgagat cgtgccatta cactccagcc caggcaacaa 13440
   gagtgaaact ccatctcaaa acaaacaaat aaacaaaaaa caacaacaac aaaaaacaac 13500
   aacaaagttt tcatttaatt ttttaacaaa ctaatctatg ttaatttgat attagtttgt 13560
   taacataaca aactaataat atgttaggca tattattcat ttatatttgt tttccttctc 13620
   ataaaacaca ttctgctctt acatttattt tcttaaatac atgaattcac caacagtctc 13680
   tttggttcct ctctactggt tcaacacatg actctttcag ctattcatta tatataaaaa 13740
   gctttgaaat ctccaactat tcttgccctt tccatctcag tgccttgctg tctactgact 13800
   ttgcagactg atgtgattcc ctctgaaaca tgaattatta ggtttttaga aaatgccttt 13860
   ttgttctttc caaagtaaaa gacaaatagg ctgggaatgt aaatttagca tttgaacaac 13920
   cattatttaa ccagctaggt tgtaatggtc aactcaggat taatgtaaaa gtgaagtgtt 13980
   gattttatgc atgccgaact cttttttgct gttaagggaa tttgtaggta agataatttc 14040
   taaactacta ttatctgtta acaaatacag tgttttatat ctaaagttta atagtatttt 14100
   aaattgtttc taattattta gcctcaccct gtgatcccac tttcatcctg ggctgtgctc 14160
   cctgcaatgt gatctgctcc attattttcc ataaacgttt tgattataaa gatcagcaat 14220
   ttcttaactt aatggaaaag ttgaatgaaa acatcaagat tttgagcagc ccctggatcc 14280
   aggtaaggcc aagatttttg cttcctgaga aaccacttat tctctttttt ttctgacaaa 14340
   tccaaaattc tacatggatc aagctctgaa gtgcattttt gaatactaca gtcttgccca 14400
   gacagccttg gggtgaatat ctgggaaaga cggccaagcc ctttatttta tgcatgggaa 14460
   ataaatgcct caatataggc ctgatttcta agcccattag ctccctcatc aatgtttttt 14520
   ctgctaaact ccaaagccct gtttctgtaa agtactttgt tgacagccct aaagcgtgct 14580
   catagcactc catggatatc caggcacttt ggaggctccc attactcaca agacttgtcc 14640
   ttcaattaac actttgtcgt attatgtggc agaaatatcc taatttaaaa gacattatct 14700
   ccttctagta gggagaatat ttgggaccat agaagctgcc aagaaacact gaatagggca 14760
   ggggtgtttg atatctcagt tgggatccta gctgatgaga tagctgggtt aggaatgaca 14820
   aaattattgg ttttatggtg tatgaaccat aaacagacat cacacttata ccctgtgctg 14880
   agctggcatg ttttattctc tgcctaaata ataattgtgt gattttatag aagtcattta 14940
   actgctctgg tgcacagttg gaatttgaag tatctttgag cccctcccac ttctaaaata 15000
   ctaattccat ttcagaggct gcttgataga aatcaatata gtagggacta gctttgtact 15060
   atcaatcagg ttgtccaaat tcttttaacc tatgctgtca tctacaaaac gtgaatgtag 15120
   taattcatgc catcttatat ttcaagatta tagagaagaa ttgtaaaaag taacagaatt 15180
   aacataaaga tgcttttata ctataaaaag gaggtctgag tctaggaaat gattatcatc 15240
   tggttagaat tgatcctctg gtcagaattt tctttctcaa atcttttata atcagagaat 15300
   tactacatat gtacaataaa aatttcccca tcaagatata caatatattt tatttatatt 15360
   tatagctgta atttacaacc agagcttggt atatggtatg tatgctttta ttaaaatctt 15420
   ttaatttaat aaattattgt tttctcttag atctgcaata atttttctcc tatcattgat 15480
   tacttcccgg gaactcacaa caaattactt aaaaacgttg cttttatgaa aagttatatt 15540
   ttggaaaaag taaaagaaca ccaagaatca atggacatga acaaccctca ggactttatt 15600
   gattgcttcc tgatgaaaat ggagaaggta aaatgtaaac aaaagcttag ttatgtgact 15660
   gcttgtgaat ttgtgatttg ttgactagtt ctgtgtttac taaggatgtt taactggtca 15720
   atcagtaatg cttgagaagc actttaagtt tttattgtat gaatgaataa caaaatagac 15780
   cactagctag actaataaag aagaaaaaag agaagaatca aatagacaca ataaaaatga 15840
   taaaggggag atcaccactg agcccacaga aatataaact accatcagag aatagtataa 15900
   acacctctat gctaataaac tagaaaatct agaagaaatg gataaattca tggacacata 15960
   cactctccca agactaaaca aggaagaagt caaatccctg aatagaccaa taacaagttc 16020
   tgaagttgag gcggtagtta atagcctaac aaccaaaaaa agtccaggac cagatggatt 16080
   cacagccaaa ttctaccagg ggcacaaaga ggagctgaca ctattccttc tgaaactatt 16140
   ccaaataata gaaaaagagg gactcctcct taactcattt tatgaggcta gcatcatcct 16200
   gatacccaaa ccgggcagag acataacaga aaaagaacat ttcaggccaa tatccctgat 16260
   gaacaccaat gcaaaaatcc acaacaaaat actgccaaac caaatccagc agcacatcaa 16320
   aaagcttatc caccatgatc aagtagcctt catccctgag atgcaaggct gtttcaatat 16380
   ttgcaaatca ataaatgtaa tccatcacat aaaaagaacc aatgacaaaa accacatgat 16440
   tgtctcaata gatgcagaaa aggcctttga taaagttcaa tacccttcat gctaagaatt 16500
   ctcaataaac taagtaatga tggaacatat ctcaaaatag taagagctgt ttatgacaaa 16560
   cccacagcca atatcatatt gaatgggcag aagctggaag gattcccttt gaaaaccagc 16620
   acaagacaag gatgccctct ctcaccactc ctattcaaca tagtattgga agctctggcc 16680
   agggcagtca ggcaagagaa agaaatgaag tattgaaata ggaagagaga aagtcaaact 16740
   gtctctattc gcagatgaca tgattgtata tttagaaaac ccctttgtct cagcccaaaa 16800
   tctcaagttg ataagcaact tcagtatagt ctcaggttat aaaatcaatg tgcaaaaatc 16860
   acaaccattt ctatacacca ataatagaca gacagccaaa taatgagtga actcccactc 16920
   acaattgcta caaagagaat ataataccta ggacacaaac aaatggaaaa acattccatg 16980
   ctcatggata ggaagaatca atatcatgaa aatggccact gcccaaagta atttatagat 17040
   tcaattctat ccccatcaag ctatcattgc ctttcttcac agaactagaa aaatctactt 17100
   tacattttat atggaacgaa agaagagccc acatagccaa gaaaatctaa gcaaaaagaa 17160
   caaaaagttc ataatcactg ggacattgga gaaatgcaaa tcaaagtccc aatgaggtac 17220
   catctcatgc cagttagaat ggtgatcatt aaaaagtcag gaaacaacag atgctggaga 17280
   agatgtggag aaataggaat gcttttacac cgttagtggg agtgtaaatt agttcaacca 17340
   ttatggaaga cagtgtgctg attcctcaag gatctggaac aagaaatacc atttgaccca 17400
   gtaatcctgt tactgggtat atacccaaag gattataaat cattctacta taaagacaca 17460
   tgtgcacgta tgtttattgc agtactattc acaatagcaa agacttggga ccaacccaaa 17520
   tgctgatgaa tgacagtctg gataaagaaa atgtggcaca tatacaccat ggaatacagt 17580
   gctgccataa aaaaggatga gttcatgtcc tctgcaggga tatgggtgaa gctggaaacc 17640
   atcatcctca gcaaactaac acaggaacag aaaaccaaac attgtatgtt ctaactcatt 17700
   aatgggagtt gaacaatgag aacacatgga cacatggagg ggaacatcaa caccggggcc 17760
   tgtcaggggg tgggggactt ggggagggat agcattagaa gaaataccta atgtggatga 17820
   caggttgatg ggtgcagcaa accaccatgg cgcgtgtata cctacgtaaa aaacctgcat 17880
   gttctgcaca tgtatcccag aacttaaagt ataaatatat aaatctaccc aaggagaaac 17940
   agaatgcaca ttccaggtga aatggaatca attactgaca aatatgaaaa cataaaatat 18000
   gccctgtgat gaagatgggt gtcattattg cacacagttt atttgagaat gggaaagaat 18060
   gacttaacct aggtgggttt cctgcattcc tgcaaattga aataggccat aaaatatttg 18120
   cagtgagagt cagaggaggg gtttcagata ggttggaatg ctctacatgg aggtgcagag 18180
   gtgggtgatg ggaaagagta ggtggatgga agagggaaag gaagccttta ttgagtgaga 18240
   acaaaaatga tttgtacaga taacagttgg aatttagtgt ctatcagata aggcgtaaac 18300
   atcaatgccg ttgtcatcgc accattgttg aaaacgctat tttttcccct attgaattgc 18360
   cttggcaacc ttgtcaaaat ccaattgact ataaatgtgt gaaaggtttt tttctgtgtg 18420
   ctctcaattg tactccattg atctatatgt ctgtcgtaat ccctatagtg caccatctta 18480
   attactgtag tattgtatta atatcaagtt attatatcaa atcaaggagt gtgagtcttt 18540
   gttgttcttt ttcaatacgt tttcactatt ctgggtccct tgaatcttca tatgaaattt 18600
   agaatacact tgtcaatttt ctcaaaggag ccaatgggga ttttgacacg attgcattaa 18660
   atcagtagat cagggaatta tgccattttt aaaattgaag tgaaattcac acaatataca 18720
   attaaccatt ttaaagtgta gacttgagtg gtatttagtg tattcacaat gttgtacaac 18780
   caccagcttt ctctggtttc aaaatgtttt tattacttca caaaaacata tcatattcat 18840
   taaataatca ctccattcct ccaacccatc atgtaattac ctctaatctt ctttctgttt 18900
   ctatggattt gcctgttatg gatatataaa ggactcacag catatgtaac ctgttatgtc 18960
   tgagtccttt cacttataat gttttagagg ctcatctaag ttgcaacatg tatcagtact 19020
   tcattctttt ttgtcgctga aatatatttt atttttccat gcacatatat cacaatttgt 19080
   tattcatctg tttatggaca tttgcattgt ttccaacctt tggctcttat agataatctt 19140
   gctttgatca tttgtgtatg tttctttatg ggcatatttt catcattctt gggtatattc 19200
   tcaggagttg aattgctggg tcaaatggta attttatgct tatcttcttg tatataagag 19260
   ccaatctttt ctacagtgaa tgtggcattt tatattcctt ccagcaatgt agaaggattt 19320
   ctattttcca catagttgct aatacttgta actttctgta tttattgaag ccatcctagt 19380
   gaatatagag tgttatctca ttgtggtttt atttatttat tcatttattt agagacagag 19440
   tcttactctg tcacccaggc tggagtgcag tggcatgatc tcagctccct gcaacttcca 19500
   cctcctgggt ttacacaatc ctcctgcctc agcccccaga gtagctggga ttacaggcat 19560
   gtgtcaccat acctggttaa ttgttatatt tttggtagag acgggttttc tccactttgg 19620
   ccaggctggt ctcgaactct tgacctcagg tgatctgccc atcttggcct ccctaagtgc 19680
   tgggattaca ggtgtgagcc actgcgccca gctctcattg tggttttaac ttgtgtttaa 19740
   tggccaatga ttttgaactt cttttaatat attattaccc atttgtatat cttctttgga 19800
   taaatgccat tcaagtacat tttctgttta aattaagttg actttatttt tctttttaag 19860
   ctgttagaat gatttatgta ttcaaaacat taaactccta catatacata atatgaagat 19920
   atttcctccc attctgttgg ttgtcatttc acattctcaa ttatattctt gctgcacaaa 19980
   gtttaatttt gatgaagttt ggtttatcta tctttccttt tgccactctg gtatcaaatt 20040
   tataaatcta ttgccaaata tgaagtcatg aagatttacc cctacatttt attctaagag 20100
   ttttatcgtt ttagctctta tatttaagtt tttccatcca ttttcagtta tatttttcat 20160
   ttggagtgaa gtaagggaga tctcagcttc attcttctgc atttcgctgt cctgttgtct 20220
   catcaccatt tgttgaagag actctttcct cagattgaat ggtctaggca cccttgatga 20280
   aagtaaattt gccatagatc tttaggttta tttcgtgatt tcagttttat tccatttgtc 20340
   ttgatgtcta tctttatgct agtaccacac tgttttgatt attacagttt gtagtaagtt 20400
   tgaagttgga aactgagtac tcactgatac agaaattaag tagaaattac ttaggcaaat 20460
   agtaagcatt tgggagtcct cagtaaggtt tttctttata ctggaaagca gccccaaatc 20520
   attttctaac aaagagcagc ttgtaaaatc gagctgcaga catacacaag gaagctggaa 20580
   acttgcacga gtgaaagctg gtagtaaaga actacctgtg accaggcaag ttcaaaatgg 20640
   cggctccttc cccacccact atgtaaatgt cacacctgat taaaccaatc tctgggccat 20700
   acgtaaatca gacactgctt cctccagcct ccctatgcaa tctgctgtgg tccacctcct 20760
   tccccctttt ccgatgtccc tctctttcac aagaagctgc ttttttctct cctttcttct 20820
   attaaacttt ctgctacata acccactcag atgtgtccgt gtcctaaatt tttctggggc 20880
   atgatgacaa acctgagggt gtatatccca gacaacgtag ctgcttcatc accatgtagt 20940
   tgcatattaa ttgaacaatc aactttccca tttctggaaa aaagaccatt ggtattgtgg 21000
   tagggattgc ctgagttttt agatcacttt gaggagtact gccatcttaa cgatattaag 21060
   tcttccaatc cctgaacatg agatagtttt caatttatgt aggtattaga tattcttttt 21120
   tcttctgttg caagggtctg gtttagttta aaaggcaagt ttatcttaaa tttttttatt 21180
   tagttatgac cattgaggat tttaaaatga taccatatga aatgccttct tttaaaaaaa 21240
   atttcaactt ttatttattt atttatttat ttatttttat tatactttaa gttttagggt 21300
   acatgtgcac tatgtgcagg tctgttacat atgtatacat gtgtcatgtt ggtgtgctgc 21360
   atccatgaac tcgtcattta cattaggtat atctcctaat gctatccctc cccactctgc 21420
   caccccacaa caggccccag tgtgatgttc cccttcctgt gaccatgtgt tctcattgtt 21480
   caattcccac ctatgagtga gaacatgcgg tgtttggttt tttgtccttg agatagtttg 21540
   ctgagagtga tggtttccag cttcatccat gtccctacaa aggacatgaa ctcatcattt 21600
   tttatggctg catagtattc catggtgtat atgtgccaca ttttcttaat ccagtctatc 21660
   attgttggac atttggcttg gttccaagtc gttgctattg tgaatagtgc cacaataaac 21720
   atatgtgtgc atgtgtcttt acagcagcat ggtttataat cctttgggta tatacccagt 21780
   aatgggatgg ctgggtcaaa tggtatttct agttctagat ccctgaggaa tcaccacacc 21840
   gacttccaca atggttgaac tagtttacag tcccaccaac agtgtaaaag tgttcctatt 21900
   tctccacatc ctctccagca ccttttgttt cctgactttt taatgatcgc cattctaact 21960
   ggtgtgagat ggtatctcat tgtggttttg atttgcattt ctctgatggc cagtgatgat 22020
   gagcattttt tcatgtgttt tttggctgca taaatgtctt cttttgagca gtgtctgttc 22080
   atatcttttg cccacttttt gatggggttg tttgtttttt atcttgtaaa tttgtttgag 22140
   ttctctgtag atcctggata ttagcccttt gtcagacaag agggttgcaa aaattttctc 22200
   ccattctgta ggttgcttgt tcactctgat ggtagtttct tttgctgtgc agaagctctt 22260
   tagtttaact agatcccatt tgtcattttg gcttttgttg ccattgcttt tggtgtatga 22320
   gacaagaagt ccttgcccat gcctatgtcc tgaatagtat tgcctaggtt ttcttctagg 22380
   gtttttatgg ttttaagtct aacatgtaag tcttgaatcc atcttgaatt aatttttgta 22440
   taaggtgtaa ggaagggatc cagtttcagc tttctacata tggctagcca gttttcccag 22500
   caccacttat taaatagtga atcctttccc catttcttgt ttttgtcagg tttgtcaaag 22560
   atcagatagt tgtagatatg tggccttatt tctgagggct ctgttctgtt ccattggtct 22620
   acatctctgt tttggtacca gtaccatgct gttttggtta ctgtagcctt ctagtatagt 22680
   ttgaagtcag gtagctttgt tcttttggct taggattgac ttgacaatgc gggctctttt 22740
   ttggttccat atgaacttta aagtagtttt ttccaaatct gtgaagaaag tcattggtag 22800
   cttgatgggg atggtattga atctataaat taccttgggc agtatggcca ttttcacgat 22860
   attgattctt cctacccatg agcatggaat gttcttccat ttgtttgtat cctcttttat 22920
   ttcattgaac agtggtctct agttgtcctt gaagaggtca ttcacatccc ttgtaagttg 22980
   gattcctagg tattttattc tctttgaagc aattatgaat gggagttcac tcatgatttg 23040
   gctctctgtt tgtctgttat tggtgtataa gaatgcttgt gatttttgca cattgatttt 23100
   gtatcctgag actttgctga agttgcttat cagcttaagg agattttggg ctgagatgat 23160
   ggggttttct agatatacaa tcatgtcatc tgcaaacagg gacaatttga cttcctcttt 23220
   tccttattga atacccttta tttctttctc ttgcctgact gccctggcca gaacttccaa 23280
   cactgtgttg aataggagtg gtgagagagg gcatccctga cttgtgccag ttttcaaagg 23340
   gattacttcc agtttttgtc cattcagtat gatattggct gtgggtttgt cataaataga 23400
   tcttattatt ttgagatacg tcccatcaat acctaattta ttgagagttt ttagcatgaa 23460
   gagctgttga attttctcaa aggccttttc tgtatctatt gagataatca tgtggttctt 23520
   gtcattgatt ctgtttatat gctggattac atttattgat ttgcctatgt tgtaccagcc 23580
   ttgcatccca gggatgaagc ccacttgatc atggtggata agctttttga tgtgctgctg 23640
   gattcggttt gccagtattt tattgaggat ttttgcattg atgttcatca gggatattgg 23700
   tctaaaattc tctttttttg ttgtgtctct gcccggcttt ggtatcagga tgatgctggc 23760
   ctcataaaat gagttaggga gtattccctt tttctattga ttggaatagt ttcagaagga 23820
   atggtaccag ctcctccttg tacctctggt agaattcggc tgtgaatccg tgtggttctg 23880
   gacttttttt ggttggtaag ctattaatta ttgcctcaat ttcagagcct gttattggtc 23940
   tattcagaga ttcaacttct tccttgttta atcttgggag agtgtatttg tcgaggaatt 24000
   tatccatttc ttccagattt tctagtttat ttttgtacag gtgtttatag tattctctga 24060
   tggtagtttg tatttctgtg ggatcggtga tgatatcccc ttcatcattt tttattgcgt 24120
   gtgttggatt cttctctctt ttattcttta ttagtcttgc taacagtcta ccaattttgt 24180
   tgatctttta aaaaacctgc tcctaaattc attgattttt tgaagggttt tttgtttcca 24240
   tgtaattgag tttccattgt ttccatggaa tgcaaaaatt ccatgttttg agtgagtttc 24300
   ttaatcctga gttctagttt gattgtgcta tggtctgaga gacagcttgt tataatttct 24360
   gttcttttac atttgctgag gtgtgcttta cttccaacta tgtggtcaat tttggaatag 24420
   gtgtggtgtg gcgaaaagaa tgtatattct gttgatttgg ggtggagact tctgcagatg 24480
   tctattaggt ctgcttggtg cagaactgag ttcaattcct ggatatcctt gttaactttc 24540
   tctctcgttg atctgtctaa tgttgacagt ggggtgttaa tgtctcccat tattattgtg 24600
   tgggagtcta agtctctttg taggtctcta agcacttgct ttattaatct gggtgctccg 24660
   ttattgggtg catatatatt taggatagtt agctgttctt gttgaattga tccctttacc 24720
   tttatgtaat ggccttcttt gtcccttttg ttctttgttg gtttaaagtc tgttttatca 24780
   tagagtagga ttgcaacctc tgcctttttt tgttttccat ttgcttggta gatctttctc 24840
   catcccttta ttgtgagcct atgtgagtct ctgcatgtga gatgggtttt ctgtgtacag 24900
   cacaccgatg ggtcttgact ctttatccaa tttgccactc tgtgtctttt aattggagca 24960
   tttaacccat ttacatttaa cattaatatt gttatgtgtg aatttggtcc tgtcattatt 25020
   atgttagctg gttattttgc tcattagttg atgcagtttc ttcctagcct tgatgatctt 25080
   acaatttggc atgcttttgc catgggtggt accagttgtt cctttccatg tttaatgctt 25140
   ccttcaggag ctcttttagg gcaggtctgg tggtgacaaa atctctcagc atttgcttgt 25200
   gggtaaagga ttttgtttct ccttcactta tgaagcttag tttggctgga tatgaaattc 25260
   tgggttgaaa attcttttct ttaagaatgt tgaatattgg cccccactct cttctggctt 25320
   gtagagtttc tgccgagaga tcagctgtta atctgatcgg cttccctttg tgggtaaccc 25380
   ggcctttctc tctggctgcc cttaacattt tttccttcat ttcaactttg gtgaatctga 25440
   caattatgtg tcttggagct gctcttctcg aggagtatct ttgtggtgtt ctctgtattt 25500
   cctgaatttg aatgttggcc tgccttgcta gattggggaa gttctcctgg atagtatcct 25560
   gcagagtgtt ttccaacttg gttccattct cctcgtcact ttcaggtaca ccaatcagat 25620
   gtagatttgg tcttttcaca taggcccata tttcttggag gctgtgttcg tttcttttta 25680
   ttcttttttc tctaaacttc tcttctcact tcatttcatt catttcatct tccatcactt 25740
   ataccctttc ttccagttga tcaaatcggc tatagaggct tatgcattca tcacgtagtt 25800
   cttgtgccat gattttcagc tccatcaggt cctttaagga cttctctgca ttggttattc 25860
   tagttagcca ttcatataat cttttttcaa ggttttttac ttctttgcca taggttcaaa 25920
   cttcctcctt tagctcagac tagtttgatt gtctgaagac ttctctcaac tcatcaaagt 25980
   cattctccat ccaactctgt tccattgctg gtgaggagct gtgttccttt ggaggaggag 26040
   aggcactctg atttttagaa tattcagttt ttctgctgtt ttttccctgt ctttgtggtt 26100
   tcatctgcct ttggtctttg atgatggtga catacagatg ggattttggt gtggatgtcc 26160
   tttctgtttg ttagctttcc ttctaacagt caggaccctc agctgcagtc tgttggagtt 26220
   tgccagaggt ccactgcaga ccttgtttgc ctgggtatca gcagcggagg ctgcagaaca 26280
   gcggatattg gtgaacagca aatgttgctg cctgatcgtt cctctggaag ttttgtctca 26340
   gaagagtacc cagccttgtg aggtgtcagt ctgcccctaa tggggggtgc ctcccagtta 26400
   ggctacaagg ggatcaggga cccacttgag gaggcagtct gtccattctc aggtctcaag 26460
   ctttgtgctg ggagaaccac tattctctcc aaagctgtca gactgggaca attaagtctg 26520
   cagaattttc tgctgccttt tgtttggcta taccctgacc ccagaggtgg agtctacaga 26580
   ggcaggcagg cctccttgag ctgcggtggg ctccacctgg ttcgagcttc ccagccgctt 26640
   tgtttaccta ctcaagcttt ggcaatgggg ggcacccctc acccagcctc gctgctgcct 26700
   tgctgtttga tctcagactg ctgtgttagg aatgagtgag cctcagtggg cataagaccc 26760
   tctgagccag gcacgggata taatctcctg gtttgccatt tgctaagacc atcagaaaag 26820
   cacagtatta gggtggaagt gaccttattt tccagatgcc ttctgtcctt ggctaagaaa 26880
   gggaattctc tgaccccttg cacttcccag gtgaggtgat tcctcaccct gcttcagctc 26940
   aggctccatg cactgcaccc actgtcctgt acccactgtc tcacaatccc cagtgagatg 27000
   aacccagtac ctcagttgga aatgcaaaaa tcattcatct tctgcatcgc tcatgctggg 27060
   agctgtagac tggagctgtt cctattcagc catcttggct ccaaatgttc tcaactttta 27120
   ttttgaagtg cacgttcatg tgtcttacat aggtatattg tgtgatgctg aagtttaggg 27180
   tacaaataat gccatcacac aggtagtgag actagtaccc aataggtagt ttttcagccc 27240
   ttgcctttat atctctctac cctctatagt aattccttgt gtattttttc catctttgtg 27300
   tcccactttt atatgagaac atgtggtatt tggatttctg ttgctgcatt aattccctca 27360
   agataatggc ttccagctac atctatgttg ctgcaaagac atgatttttt ttaatggctg 27420
   catagcattt catggtgtat atacaccaca ttttgtttat gtgacacatg attggttggt 27480
   tctatgtctt tactattgtg aatagtgcag gaatggacat actagcacgt gtcgttttgg 27540
   tagaacaatt tgttttcctc tgggtacata ctcagtagtg gaattgctgc atcgaatggt 27600
   agttctgctt ttagttcttt gagaaatctc aaaactggtt tccatagtgg ctgaactaac 27660
   ttacattccc accagtagta tataagtgtt ctgttttctc tgcagtctca ccaacatctg 27720
   ttattttttg gctttttaat catatccatt ttgattggca tgagatgata tctcattgtg 27780
   gttttgattt gcatttctct gatgattagt gatgttgagt gttttctcat atgcttgttg 27840
   gccatgtgtg tgtcttcttt ggaaggacat atgtcctttg cccacttttt aatggggtta 27900
   cttgtttttt aacttgttga attgtctaac ttccttatag attctcgcta ttgaaacatt 27960
   gttggattca cagtttgcaa atattttgtc ccattctgta ggttgtctgt ttattctgtt 28020
   gatagtttct cttgctgtgc agaagctctt taattaggtc ccacttgtca atattcattt 28080
   tggttacaat tgcttttgag gagttattca taagttcttt gctaaagcct gtatccagaa 28140
   tgttatttta tagatatttt tccatcagat tcttatagtt tgaggtctta aatttagatc 28200
   attaatccat ttcgagttaa ttattgtata tggttcaagg aagtgtttca gtttcattct 28260
   gcatatggct agtcagttat cctcattcaa tttactaaat agagaattct ttcctcattg 28320
   attatttttt gtcaactttg ttgaagatca gatggctata ggtgtgtggc tttatttctg 28380
   gcttctctac ttctgttcaa tttgtgtttt gtaccagtta catgctctct tggttactgt 28440
   agcttacagt atagtttgaa gtcaatgtga agcttcccac tgttcttttt gtttaggatt 28500
   gctttggatt tgggctcttc tttggttcca tatgaatttt agaataaaat aatattggta 28560
   gtttgatagg aatagcattg aatctgtaga tagctttggg cagtctagcc attttaatga 28620
   tattgattgt ttcaatccat aagcctgaac tgtttttcca tttgtttgtg tcatctatga 28680
   ttttgttcat cagtattttg tagctctcct tgtagagacc tttttgcccc ctttgttagt 28740
   tgtattccta ggttttttct tgtggctatt gtaaatggga ttgagttctt gattggcttt 28800
   caggatgaac atttttggtg tttaaaaatg ctacagattt ttgcatattt attttgtgta 28860
   ctgaaatctc actgaagttg tatgtcagtt ccaggagttt ttggaaagtt ctttagggtt 28920
   ttctaggtct gaattgggtc ttctaggtct gagatgggtc ttctacttct actagtagga 28980
   gatgggcctt gcaatcttga agacagtaga aggatgggtc ttatttttaa tccaacttcc 29040
   tactctttgc cttttatgtg gagcatttag actttatctt caatgttaac actgatatat 29100
   gaggttttga ttctattgtg aagttgttag ctggttgttt tgtagtttct attgtgtgat 29160
   tgctttatag agtctgtggg ctatatactt aagtgtgttt tgtggtagca ggtattgttc 29220
   tttcacttcc atatttagaa ctcccttaat gatctcttat aaggctggtc taatggtaac 29280
   agattttcct ggtagttact tgtttggaca agattgtatt tcttccttga ttttgaagca 29340
   tagtttggca aaatataaaa ttcttttttg taatttttat taaaatgaat gttgaaaata 29400
   ggctgagcat ccttttagct tgacaaggtt ctctttgtat gtgatctgat ttttttctct 29460
   agctgccttt aaaattattt tttagccttg actttggaca gcctggtgag taaatgcctt 29520
   ggtgatgtta attttgtata gtgtctcaca tatattccct gggtttattg tatttggatg 29580
   tctacctttc tagcaagttt gggaaaattt tctcaactta ttagcccaaa tgtattttct 29640
   agattgttta ctttttcttc tctctcagga atgccaataa ttcctaggtt tactcctttt 29700
   aaataatcag atatttcttg aagactgttc atttattaaa attatttttt ctttattttt 29760
   gtctgacttg gttagttcaa aagactggtg ttcaagctct gaaattggtc cagtctattg 29820
   ataaacgctt caattgaatt ctgaaattcc ttaagtgact ctttcaattc cagaagcttt 29880
   gattgatttc tttttaagat gtttacttct cgctccattt cctggactgc tttagtagtt 29940
   tgtttgcatt aattttcaac cctgccttgg atcactgagc ttacttgcag tccatatttt 30000
   gaatagttta tctgtcattt ctgagttgcc agtttggtta gggttcattg ctggggagtt 30060
   catgtgatcc tttggaggtt ttacgacatt cagacatttc atggtgccag aagtcttatg 30120
   ctggtttctt cttatttgga gaggctgccg cttctgattt ttgaaattat ttttgtgcag 30180
   atagaattgt ttttctctcc ctatttttgt tctttccttt ttctcttccc cttcctatgg 30240
   gatgtgacta taaagtatgt tgggtagggc ctttgacttg gattctatag ctgtgttcac 30300
   ttctgtgggt agttttatat tggcctgtgc agtttgacat acatgtcagt agatggcact 30360
   tatgagcaaa agccagctgt gactaggaca acttgatata gcacagcttg atccttgttt 30420
   tctgggggaa cttctctgtt tcctcagata atctgttcat ctgtggaatt cagagtagtc 30480
   tgggctccct gctgagcccc agggggataa agatcggtgt cactggactg ggcagtcctg 30540
   tctacaggtc ccctaatggc aggcactagc accagcactg agagacaact gatttttgca 30600
   ttgatcttgt accctgaaat tatgctgaaa tcatttatta gctctactta ttttctatct 30660
   gtattgcatg agatttttct atatagagag aatcatgttt cagtcaaata cacatgattt 30720
   gaatttttcc tttcctattt gaatgtgttt tattttattt cttgcctaat tgctctggct 30780
   agaacttcca atactatgtt taatagtagt aaaaatggac atccttgttt tttttctttt 30840
   ctttctgatt ttaagttaca agttttagtc tttcacaatt tagtatgctt tagctttgca 30900
   gttttcataa atatgcttta ttatgataaa ggagttttat ttcatttata gttttctgag 30960
   tgtttttgtt atgatgggta ttaaaagttg tcaatttttt tttctgcatc taatgagatg 31020
   aacatgattt tctcttttat tctgatatgg tgtagtgcac tgattgactt tcttgttgaa 31080
   ccacttttga atttctggga taaatctcac ttgcttattg tgagatttat ttttataatg 31140
   tttattatta taatttttta attttgtaaa aaattgttgc aaaatacaca taacataaaa 31200
   gtttctgtat taatcatttt aagtgcatag tattttggta ttaattacat tcacattgtt 31260
   gtgcaaatgt cactgccata catctgtaga attcttttca tcttgcaaaa cagaaacttt 31320
   atacccattt aataataact cttggccggg cattgtagat catgtctgta atcccagcac 31380
   tttgggaggt tgaggtgggc agattggatg agctcaggag ttcgagacta gcctgggtaa 31440
   catggcaaaa ccctgtctct accaacaaag caatcaacaa acaaaatgaa agtaacataa 31500
   tttcctcaac tgtgcaaact aattcatatt aataattcca tgaaatgtgt ccatagtaac 31560
   acaagagcag aagatgggcc ttgtaaaggt ggcccaccac attatttgag gtctttgtca 31620
   gtcttatctg ccagatactg tgtggactaa ctgcagttaa aattcccatt cccttatcac 31680
   ttgccctgaa atttatctga aatcaaaggg aatgggttgc ccaaagagaa gtgtccagag 31740
   acgtggatga tgcgacaaga aatgttgtag aaccacaccc ccacctcaaa tgataactct 31800
   acatttctcc cttccaccac cccatggcaa ccaacattct actttgtgtc tctataaaat 31860
   cgagtatgct gggtacctca taaaagtgga atcattacat atttgccttt tgtgactggt 31920
   ttatttcact tagcctaatg ttatgttctc agggttcatt catgtttttg catgtgtcag 31980
   atttttcttc ctttctaagg gtgaataata ttccgttgta tgtataaacc aagtttgctt 32040
   acccatttat ctgttgatgg gcacttggat tactttcacc ttttgacttt ttgagtaatg 32100
   ctattatgaa tatgggtaaa aaaatatctc tttgatgtgc tgctttcaat tcttttgggt 32160
   ataaataacc caaaagaata acccaccacg cctggttaat tttttgtatc tttagtagaa 32220
   actgagtttc accatgttgg ccaggctggt ctcaaactcc tgaccctgtg atccacccac 32280
   ctcagcctcc caaagtgctg gaattatagg tgtgagccac gtcgcccggc ctacatttat 32340
   tgatttgcat atgttgaacc aggcttgcat cccagggatg aagccaactt gatcgtggtg 32400
   gataagcttt ttgatgtgct gctggattca gtttaccagt attttgttga ggattttcgc 32460
   atggatgttc atcagggata ttggcctgaa attttctttt ttttgtttta tgtctgccaa 32520
   gttttggtat caggatgatt cttcccttat aaaatgagtt aggaaagagt gcgtcttttt 32580
   ctactgtttg gagtagtttc agaaggaatg gtaccagctc ctctttgtac ctctggtaga 32640
   attcggctgt gaatccttca ggtcctgggc tttttttttg cttggtgagc tattccttgc 32700
   tgcctcaatt tcagaacttg ttattggtct cttcacggat tcgacttctt cctggtttgg 32760
   tcttgggagg gtgtgtctag gaatttatcc atttcttcta gattttctag tttatttgca 32820
   tagaggtgtt tatagtattc tctgatggta gtttgtattc ctgtaggatc agtggtgata 32880
   tcccctttat catttttttg ttctctctat ttgattcttc tttcttttct tctttattag 32940
   tctggctagc agcctatttt gttaatcttt tcagaaaatt agctcctgta ttctttgata 33000
   ttttgaaggg attttcctgt ctctatctcc ttcaattctg ctctgatctt agttattttt 33060
   tgtcttctgc taggttttga atttgtttgc tcttggtttt ctagttcttt taaatgtgtt 33120
   gttagggtgt tgattttaga tctttcctgc tttctcctct ggacatttag tgctataatt 33180
   ttccctctaa acactgcttt agctgtgtct tagagattct ggtacactgt gtccttgttc 33240
   tcattggttt caaagaacat ctttattttg ccttcatttt gtcatttacc cagtagtcat 33300
   tcagagcagg ttgttcagtt tccatgtagt tgtgcagttt tgagtgagtt tcttaatcct 33360
   gagttttaat ttgatggcac tgtggtctaa taggttgttt gctttaattt ctgttctttt 33420
   gtatttgctg atgggtgttt tacttccaat tatgtggtca attttagaat aagtgtgaag 33480
   tggtgctgag aagaatgcat attctgttga tttagggtgg agagttcttt agatgtctat 33540
   tatgcccgct tggtccagag cttagttcaa gtcctggaga tcctagttaa ccttctgtct 33600
   cattgatctg tctaatattg acagtggggt gttaaagcct ccatctatta ttgtgtggga 33660
   gcctaagcct ctttgtaggt ctttaagaac ttgctttatg aatctgggtg ctcctgtatt 33720
   gggtgtgtat ctatttagga tagttagctg ttctcgttgc attgatccct ttaacattat 33780
   atgataccac cccttttttt aatctttgtt ggtttaaagt gcgttttatc agagactagg 33840
   attgcaaccc ctgctttgtt ttgctttcca tttgcttggt aaatattcct ccatcccttt 33900
   attttgagcc tatgtgtgtc ttttcacgtg agatgggtct cctgaataca gcacattgat 33960
   gggtcttgac tctatccaat ttgccagtct gtgtctttta attggggcat ttagcctgtt 34020
   tacatttaag gttaatattg tgtgtggatt taatcctgcc attatgatgc tagctgatta 34080
   ttttgccttt tagttgatgc agtttttttt atagtgttga cggtctttac aaaatttggt 34140
   atgtttttgc agtggctgtt cccagttgat cctttccata tttagtgttt ccttcaggag 34200
   ctcttgtaag gcaggcttgg tgatgacaaa atccccttgt ggtgatgggc ttccctttgt 34260
   ggtaacccga cctttctttc tggcttccct taacattttt cctttctttt aaccttggtg 34320
   aatctgacaa ttatgtgtct tggggttgcc cttcttgagg agtatcattg tgggtgtttt 34380
   ctgtatttcc tgaatttgaa tgttggtctg tcttgctaga ttgtggaagt tctcctggat 34440
   aatatcctga agaatgtttt acaacttggt tccattctcc ccatcacttt caggtacacc 34500
   aatcaaacgt agatttggtc ttttcacata gcgccacatt tcttggaggc tatgttcatt 34560
   tctttttatt cttatttttt aacttttttc ttcacatttt atttcattaa gttgaccttg 34620
   aatctctgat atcctttctt ctgctttatc aatttggcta ttgatattta tttatgcttc 34680
   acaaagttct cattctgtgt tcttcagctc catcaggtca ctcatgttct tctctagtta 34740
   gcaattcgtc taacattttt tcaaggttct tagcttcttt gcaatgggtt agaacatgct 34800
   cctttagctc agtggagttt gttattacac accttctgaa gcctacttct gtcaattcat 34860
   caaattcttt ctctgtccag ttttgtcccc ttgctggcaa ggagttgtga ttctttggag 34920
   gagaagagat gttctggttt tttaaatttt cagccttttt gtgctgtttt tttttttctc 34980
   atatttgtgg atttatctac ctcttgtctt tgatgttggt gacctttgaa tgggtttttt 35040
   gcgtggacat cctttttgtt gatgttgatg ctattccttt ctgtttgcta gtttttttcc 35100
   taacagtgag gcccttctgc tgcaggtctg ctggagtttg cttgaggtcc actccaaacc 35160
   ttgtttgact gggtatcacc agaggaggcc acagaacagc aaagattgct gtgtgtttat 35220
   tcctctggaa gcttcatcct agggggacac ttgccagatg ccagccaaag ctcttttgta 35280
   tgaggtgtct gttgacccct gctgggaggt ctgctgttct ctttagagcc agcaggcagg 35340
   aacatttaag tctgccgaag ctgcacccac tgccacccct tcttccaggt tctcagtaca 35400
   gtgagatggg agttttatct ataagcccct gactgggact gttgcctgtc tttcacagat 35460
   gcaactgctc agagagtagg aaactagagg ggcagtatgg ctacagtggc tttgcggagc 35520
   tgaagtggac tccacctagt cctagcttcc tggaggcttt gtttacactg tgaggggaaa 35580
   accacctact caagcctcaa caatggcaga ctcccctcac ccccccaccc cccccaagct 35640
   cgaacatccc cagtcaactt cagactgctg tgctggcaaa cagaagttca agccagtgga 35700
   tcttagcttg ctgggctcca tgggggtgag atcccctgag ctagatctct tggctccctg 35760
   gcttcagtcc tctttccagg ggactgaatg gttctgactt gctggtgtta cagcaccact 35820
   ggggcataaa aaaacccctc cagctatctt ggtgtctgcc cagatgacca cccagttttg 35880
   tgcttgaaac ccagggccct ggtggtgtag gctctgaagg gaatcttctg gtctgtgggt 35940
   tgtgaagacc atggaaaaac tgcagtattt gggccagagt gcaccgttct tcacagcaca 36000
   ttccctcaag gctttccttg gctatgggag ggagttcacc aatcccttgg gcttccaagg 36060
   tgagacaaca ttcctccctg cttcagttta gcctccatgc actgcaccta ctgtctaaac 36120
   attcccagtg agatgagcca ggtacctcag ttggaaatgt agaaatcacc taccttcttc 36180
   attgatctcg ctgggagctg ctgactggag cttttcttat tcagtcatct tgccagccac 36240
   cctcctgatt gagtttcttt aaggtaatta ttttgaattc cttttttgaa tatttgtgga 36300
   ttttttttca ttgaggtctg ttatttgaga gttattatgt ccctttggtg gtgccatatt 36360
   tctttatttt tcatgttgtg tctctgaatt tatgtctatg catgtgatgg aacaattgct 36420
   attccaaaat ctctagagtg aatttcatag agaaagactt ttatctgaag ttgagtgttt 36480
   gtgtgcaggt gacgaagagt gggtaactca gtttttgata gatgcattgt gatagtatct 36540
   gtgtagtttc tctagctatg gccaatatca gcaataattt tgggtgcctc agtggcctag 36600
   gctgtagaag tttgcagcag tatcaggagc agtgtaggtt gttaatgtcc ttagtgtcaa 36660
   agtatttggg cgtcctccta tttttatttt cctaaaattg gggtgactta gcctaaggga 36720
   ttccttttgg tgtcatctct gacatggcat taactcagcg gcattggtgc tgggttacaa 36780
   gtacagatcc ttgaagttgt catagaagca tcattctaga ctcagggtct tttaatcatg 36840
   tattgtgaca cctgggtctt gcggtgcagg ttcactctct gtggcagtgt tgaatgcaga 36900
   ttacccacag aaccaaggtc tatgactcta gcacacctta gcagctgaag cccacagact 36960
   gagttgtggc tgtgaatctg tttctgaggg tgaggtgcag gaaaatgccg tggcctgcct 37020
   ctgatgtaga aggggtgctc tttagatttg agcccagcaa gtagaatatg gctactatca 37080
   ttaataagat aggatatact accttattaa tgctaagtaa ccaagctcaa gtcaagagat 37140
   ggaaatgtta atactggttt gtgactgcac tgttcttctt acttaagagt aattttcaac 37200
   atggcaagct tagaaaactg tgtgaagctg atgttaccaa gggagaggac tgtctttgtc 37260
   tacacatgtt gagagaatct agtctatttc agtggtgttg attacacaat attcaacatg 37320
   acacacatca cttggagact ctaaaagcat cattaattta ttgctgctgg ccttggggct 37380
   ggttaaaaag taatacgttt tttatattga tgatctcttt caactaagta ttccttatcc 37440
   attgctttct ttctcccaac ttccaaatta tgagaactcc agtctctaag ccttggcata 37500
   tgcagtatcc ttactctctc ctttatcccc tgtggcaatt aatcccttta ttctccatct 37560
   tcatcattgt tctttctctt cactttgtat atttttgttt attcctgcac tactgcaccc 37620
   tagaacaagt tgtctgttgc ctcccatcta gattataata gtgtgggccc atcttgtcat 37680
   tgtaaaccca ttctcttcct ctataattta tcttaggttt tgctgtcata ttgctttttt 37740
   aatatttgtt tttacaaaat ttcaacttta taatgaatct gggggcaata ttgagtccat 37800
   gtacctttct gacagaaaaa tataattgca gactcttgat tcaggctcac agtgtctaat 37860
   gatatggccc caatttacgt tttctattaa tttagattgt ggttgtctac aggtaataat 37920
   ggctttaaca cataatgtct ttatttatct caaatacagg gattctcttg gtggggcaat 37980
   ctaggataat acagcagccc caaagcagca gagagtcaag tgccttcctt tttcccacta 38040
   aatttagtat acagaccttc attttgatga ttaaatccca ggatggggtc tactctacct 38100
   cctacttcac atttgtgttt tgagtatgaa agaggaaata gaggaaatgg acctagagac 38160
   cttctgctta tatattttag caaaaaaaaa tatgctgtgt gactcagcta gctgcaaaga 38220
   gcctgatgaa tggaattttt aggcaagcat ggaataaggg agtaggaaat aaagtttggg 38280
   caagttggtc tacagcctct gctatacaag cagtattttt tttctagtac tgtactttcc 38340
   agtttctatg ttggtaacta tataactatg tgaataattt tgaattcact gtaatcaaat 38400
   atgctggtaa ataatttgtc agataattgc atcaaatcat tcctaggaaa agcacaacca 38460
   accatctgaa tttactattg aaagcttgga aaacactgca gttgacttgt ttggagctgg 38520
   gacagagacg acaagcacaa ccctgagata tgctctcctt ctcctgctga agcacccaga 38580
   ggtcacaggt atgatcagag atgataagtt aattaatttt cagaaaagat tttgggaagg 38640
   tgttgctagt gtcctccttc ctgtttctct tagagaagct tcattattta aacttttgtg 38700
   cttccagctg taatctgttt caaactaatg gtgattacaa tgggatatct tggcctggca 38760
   tggtggctca cacctgtaat cccagcactt tgggaggctg aggtgggtgg atcacttgag 38820
   gctaggagtt caagaccagc ctggccaaca tggccaaatc ccatctgtag taaaaataca 38880
   aaacttagcc aggcatggtg gcacatggct gtaatcccag ctactcgagg ggctgaggca 38940
   ctggaatcaa ttgaacccag gtggtggagg ttgtagtgag ccaagatttt gccactatac 39000
   tccagtctgg gcaacagagt gagactctgt ctcagaaaga aaaaaaaaga aaaaaagaaa 39060
   aagaaaagat aaaaggggca tctttgcaca gtagaggaag ataactgaga gaaatgaaga 39120
   cagcatggca gtagcaaaag cagtagaact ctggtccaat gtgtctggat ttatggcagg 39180
   aagagacaag ataaattggc ctgggattgc atgttggttt tattatgaga aggccatttt 39240
   aaatagcaag tatattcttc aagataactt ttctcattct caaaatttca ggttcgaatg 39300
   ctggagtagg gaaacttgaa actctcctta ttgaaggata aatggtaatc ctaaaaatgt 39360
   agtgatctgc ctgagaaaat tcctagtcca ataagtactc taaaaagtta gattcataag 39420
   aaaggtgatt ctgtttacta gaagaggtat ataaagaatg ttccatttag gcagaatatg 39480
   tacctgagac agtttccatg aaactgttgt tgggcaaaat aagatttttt tgaggaagtc 39540
   aataatttta tcttttatga acaactttac tttagaaatt tacttttaag gacttttggt 39600
   tatgctgcag ataagaaata ttcttttttt ctcctatgtc agtatccccc attgaaatga 39660
   caataaccta attataaata agaattaggc ttttttttga acagttacta gcctatagag 39720
   ttctagaaga ttttttgtca aattttatta tagattaaag ggtacaaata caggtttgtt 39780
   acataggtaa gttgtgtaac actgaggctt ggggtcccag ctattccatc acactgacag 39840
   taaacatatt acccaacaga tggttcttca gcccacatct cttctctccc tcccccatct 39900
   agtgatcctc agtgtctatt gttcccatct ttatgttctt gtgtattcaa tgcttacctc 39960
   ccacttataa gcaagaacat gtggtatttg gttttctgtt cttgtattag attgcttagg 40020
   ataatggcct cctgcttcat ctataatgct gcaaaagaca taacttttgt tctttcttat 40080
   ggctgcataa cattccatta tgtatgtact agtctgttct catgctgcta ataaagacat 40140
   acctgagaca gggtaactca taaaggaaag aggtttaatg gactgtcaat tccacacggc 40200
   tggaaaggcc tcacaataag tctctgatag agacttattc actatcacaa gaatggcatg 40260
   ggaaagtcag gcctccatga ttaaattacc tcacattgag tacctcccgt gacgtgtggg 40320
   aattatggga gctacaattc aagatgagat tttggtgggg acacagccta accatatcag 40380
   tgtatatttg gtacattttc cttatacaat ccactgtcga tgggcacata agttgattcc 40440
   atttctttgc tatcgtgaat agcactacat attggggtga acatattggg gtgtgtgtct 40500
   tattgataga atgaatattt tgggggcata tactccatag tgggattgct gggtcaactg 40560
   gtagttctat tttaagttct ttcagaaata tccaaactgg tttccacagt ggctgaacta 40620
   gtttgcatac ccaccaacag tgtaaaagca tttccatttc tctgcaacct caccagcatc 40680
   tgttgttttt ggacttttta atattaataa ttgctattct gactggtgtg agatggtacc 40740
   tcattgtgat tttgatttgc atttctctga tgattagtga tgttgagcat tttttcataa 40800
   gtttgttggc tgcttgtatg tcatctattg agaagtggct gttcatgtct tttgcccaat 40860
   tttaattgat tttttttgtt tttttttttt gcttgttgat ttgtttaagt tttttataga 40920
   ttttgtctgt tgatacatat ttagattttg gatattagac atttaggtcc tttaaggtag 40980
   gaagagtaca ggaaatagtc aagaagactt taacaaattt ctaaaaacag agagcatata 41040
   gaggaatgat atatctgtac agagagggta aacttataac caaaaggtca gaagagaaaa 41100
   atttatcaag gatgaaaagt taatcagtgt cccttaaatc ctcccaatat tggtatttgg 41160
   aagtaccgag ggtcaaagat ggtggagaat gaggctgaaa aaggaggctc tccctgccta 41220
   cgtttcctcc acaactcttt tcatcaggta actgatttcc ttccacaaga agaaggaggc 41280
   tcactctcta gagaaactga gcttaagaag ccctgtactc agagacacac agcagtgcca 41340
   gaaaaggcat gaggtgtagg ctagaaagac agattaggta cacatccact ccttgaattc 41400
   agagactctt agctctttta ttcatcttcc ttcctaaaat tcagcagcta gctctatcct 41460
   acagaaaggg aactgatcta gagaaatggc atttttcttt tgccatctta acagacaatt 41520
   ctcttcaatg tccaacagag tgaatgaaat gaaaacatat actgctagat acagggttat 41580
   ggaatttcag gacatgagaa ttaaagggaa aatcacaaaa actttcagag ctgtaccagg 41640
   ttatattgaa agaattagaa ttcagaattc tcaagaagaa ctattaatat tatcttgtat 41700
   attaataaaa atagtgtttt cagatgtgca tgtttgccaa aaatttttac cattttccct 41760
   ttctcagaaa aatactgaag aatgttcttc attcaaatgc ctgaaacctt gttagagaaa 41820
   gtcctgagct ccagcaatca ggaaacctcg tgtaggagaa tgagagtgtc agggaggctg 41880
   cctagcacca gacctagaaa acaactactt cagaacagac ctgggggata attcagaagt 41940
   gagatgagac agcaagaaca aaagagatgt gacagatttt atgagagttt ccaataacta 42000
   aaaaggagtt tcaaacatct gctggaacat ttgtaaataa ttctaacttg ggaaatggaa 42060
   aattacaaat tgaaataaag agacattgtt aagcccagga aagtacaaaa ataaaattta 42120
   atcatagttg tcttaccaga gaaccatatt tgtgtagtca gaatgatgta agcaatgaat 42180
   actgatatca taaaaattgt catctaaata tattctggaa aaagcagtgt ggaaaagtga 42240
   gtgtgtctat ctgagggtgt gttcaaatat gaggggaaga tggagattaa aattctcatc 42300
   tctcttaata aaaaattatt aaatataatc tacaaaaaga taagtaagaa ttagaaacag 42360
   agtatttaaa aatgtgaatg tatgttcaga agaagctgct agaatgttca aaatggtaga 42420
   tttggaaagt gtgagaatga agagaatacc attgtgattt gtgacccctc ccacacacag 42480
   atgcaccaat acagtcatat atatgcataa atctgatagt ataatttaac gtttaagcaa 42540
   catatgttat tttgatatga aattaatctg tttttcaaag acagtaattt catgactaaa 42600
   ttatatgctt actttatgtt ttcaaacatt ttattatttt tatttgtatt aattatcata 42660
   tatactaatg tatatgtata tacatatctt ttaatttttt ttaattttta tgggtacaaa 42720
   gtaggtatat atcaatatgt ttgggggtac atgagatgtt ttgatacagg catgcaatgt 42780
   gaaataagca tgtcatggag aatggggtat ctattccctc aagcatttat tctttgagtt 42840
   acaaacaatt caattacact ctttaagtta ttttaaaacg tacaattaag ttattattga 42900
   ctatagtcac ccttttgtgc tatcatatag tagatcttat tcatctccac ctcccctccc 42960
   cccacactac cctttcgagt ctctggtaac catctttcta cactttatgt ccattagttc 43020
   aattctttta tttttagatc ccacaaataa gtgagaacat gctatgttcg tctttctgtg 43080
   cctggcttat ttcacttaac gtaatgatct ccagttccct tcatgttgtt gcaaatgact 43140
   gaatctcatt ctttttatgg ctgaatagaa ctacattgcg tatatgtact acattttctt 43200
   tatacatttg tctgttgatg catatttagg ttgtttcaaa gtcttagcta ttgtaaacag 43260
   tgctgcaaca aacaagagtg cagatacctc ttcgatatac ctatttcctt tcttttgggt 43320
   atataccaag cagtagggtt gttggatcat atggtcgctt tatttttagg attttgagga 43380
   acatttaaac agctctccat agaggtcaca ctaatttgca ttcccaccaa cactgtacga 43440
   gggtccactt atctccacat cctcactagc atttgttatt gcctgtcttt tggatataag 43500
   ccattttaac tggagtgaga tgacatctca ttgtagtttt gatttatatt tctctgagga 43560
   tcagtggtgt tgaccacctt tttgtatgcc tgtaacccat ttatatatct tcttttgaga 43620
   aacatctatt caaatctttt gcccactttt ggattggatt attagatttt ttttctatag 43680
   agttgcttga atttcttata tattctgatt actaatctct tgtcagatga atagtttgca 43740
   aatatttctc tccattctgt ggattgtctt ttccctttgt tgattgtatc tttggctgtg 43800
   cagaagcttt ttaacttgat gtgatcccat ttgttcatgt ttgctttgaa tgcctgtgct 43860
   tgtagggtat tgctcgaaaa atttttgcct agaccaatgt cctggagatt ttccctagtg 43920
   ttttcctata gtagtttcac agtttgaggt cttagactta agtctttaat ccattttgag 43980
   ttgatttttg tatatggcaa gggatatgag tccagtttca ttcttctctg taggatatta 44040
   aattttccca gcacatttat tgaagagact gtctttcccc cagtgtacgt tcttggcacc 44100
   tgtgttagtc tgttctcatg cctctaataa agccatacac aagactgggt aatttataaa 44160
   ggaaagaagt ttaatggact cacagttcca catggctgga gaggcctcac aatcatggca 44220
   gaagatgaat gaggagaaaa gtcatgtctt acgtggtagc aggcagatag ctttgcaggg 44280
   gaactcacat ttataaaacc atcagatgtt gtgagattta gtcactatca tgagaaaaat 44340
   atggaggaaa ccacccccat gattcaatga tccctacctg gcctccccct tgacttgtgg 44400
   ggattatttc aattcaagat gagatttggg gtggggacac agccaaacca tatcagcccc 44460
   tttgtcaaaa atgagttaac tctagatacg tgaacttgtt tctgaattct ctattctgtt 44520
   tcattggtcc atgtgtttgt ttttatgtca gtaccatgct gttttagtta gtatagctct 44580
   gtagtttaat ttgaagtgag ataatgtgat tcctccagtt ttgttccttt tgcttaggat 44640
   agttttgact attctgggtc ttttgtgggt ccatatacat tttaggattt attttttcta 44700
   tttctgtgaa gaatgccatt gatattttga taggaatttt attgaacctg tagattgttt 44760
   tgatagtatg ggcactttaa caatattgat ttttccaatc catgaacatg aaatattttt 44820
   ctgtttgttg ggtcctctta aatttctttc ctcagtgttt tatagttttc attatagaga 44880
   tctttcaatt ctttggttaa attaatccta ggtatttaaa attatgtatg gctattttca 44940
   atgggattat tttttaaatt tctttttctt ttttttcact gttggcatat aacaacacta 45000
   ctgattttta tgttgattgt gtatccttca actttactga atttgttcat cagttctaat 45060
   aattatcttg tagaatcttt agtttttcct actataaggt tatatcatct gcacacaagg 45120
   atattttgac ttcttccttt ccaattttga tagcctttat atctttctct tgtctgattg 45180
   ctcttgctag gtcatccagt actatgatga ataacaatgg tgacaatggg catccttgtt 45240
   gtgttcctga tcttacagga aagacttcct attttctcca ttcaggatag tactagttgt 45300
   gggtctgtca tatagctttt ttatgttgag gtatgttcct tctatctcaa gtttttttta 45360
   tgaattttca tcaagaaggg atgttaaatt ttatcaaata ctttttcagc atcaactgaa 45420
   atgatcatat ggcttttatc cttcattctg ttgatatgat gtatcacact gcttgatttg 45480
   cacatgttga accattcttg catccaaggg ataaatccca cttggtcatt gtgaatgacc 45540
   tttttattgt attgttgaat ttggtttgct agtgttttgt tgaagatttt tgcattagtg 45600
   ttcatcaggt gtattggcct gtagtttttt tttttttttt tttttttttt gatgtgtctt 45660
   agtctgattt tggtatcagg gtaatactgg cctcatagaa tgagtttgga agtattctct 45720
   tctcctttat tttttggaat agtttgagta gggtcggtat tagttcttct ttaaatgttt 45780
   ggtaaaattc agcagtgaag ctagccagtc ttgggatttt ctctactggg atgattttat 45840
   ttctcgtttg atctttgtgc tggttattgc atgttcagat tttggatttc ttcctggttc 45900
   aatcttgatg ggtcatattt gtgtagggat ttgtccattt cttctagatt ttccaattta 45960
   ttggcatata gttgctcata gtaaccaata attattcttt gaatttctgc agtatcagtt 46020
   ctaatgcctc ctttttaatt ttttatttat ctgaatctac tctttttttt tttcttagtc 46080
   tggctaacgg tttgtcaatt ttgtttaact tctcaaaaaa gcaacttttt gtttcattga 46140
   tcttttgttt tgttttcttc acttaaattt tatttatttc tgctctgatc tttattattc 46200
   cttttcttct actaattttg gttttaattt gcttttgctt ttctaattct ttaggatgca 46260
   tcattaaatt gtttgtttgt caattttctc catttttgat ataagctctt gtagctataa 46320
   acttccctct tagtactggt cttgctgtaa cccctaggtt ttggtatgtt gtgtttccat 46380
   tatcatttgt ttcaagaaat ttttcaattt tctttttaat ctcttcatgg gtccactgtt 46440
   catttatgag catattttta aatttccatt tatttgtgta gtttccaaaa ttcctcttgt 46500
   tattggttgc tggttttatt acattgtggt cagagaagat gcttgatatt atttcagttt 46560
   ctttgaatat tttaagactt gttttgtgac ctaacatacg gtcaattctt gataacaatc 46620
   catgtgctgt ggaaaagaat gtgtattctg tagcagttgg ataaaatatc ctgcaaatat 46680
   ctatgagatc catttgatct atagtgcaga tgaatttcaa tgtttccttg ttgattttct 46740
   atctggatga cctgtccaat gctgaaagtg gggtgttgaa gtctccaggt attattatat 46800
   tggggcctat ctctctctag ttctaattat atgtctttta tatatctggg tgctgcatta 46860
   ttggttgcat atatatttaa acttgttcca tcttcttgcc aagctgacca ctttatcacc 46920
   aatagtgatc ttctttgtgt ctccttatgg tttttgtttt gaaatctact ttgtctgttt 46980
   taaatatagt aactcatgct ctttttttca tttccattgg caggtactgt ctcattcaat 47040
   tcctttattt tcagcctatg tgtgtcttta taagtgaagt gtgtttcttt taggcaacag 47100
   attaataggt cttgtttttc catccaggtc agtaacaggt cagtatgtct tttgattgga 47160
   gattttattc catttacatt cagtgttatt attgataagt aaggacttac ccatgcccct 47220
   ttgttatttg ttttctggtt gttttgtgga cttctcttcc ttctttcatt tcttcctgtc 47280
   ttcctttatt gaagagaatt ttctccactt atatgtgtac agatttttct taatatctgg 47340
   tttatggcag ttacacattt gtgcatctgt aaccatcctc tctttaagtt tgcatatact 47400
   tccagcacta taatttaaat ttataatgat gtttggatac cttcatgatt catatacccc 47460
   tgaattgcta caacaaatgt gccatttttc tccttttcca tcagttttta cttgtgtctt 47520
   atcagctaaa gtccaggaag agattgaacg tgtgattggc agaaaccgga gcccctgcat 47580
   gcaagacagg agccacatgc cctacacaga tgctgtggtg cacgaggtcc agagatacat 47640
   tgaccttctc cccaccagcc tgccccatgc agtgacctgt gacattaaat tcagaaacta 47700
   tctcattccc aaggtaagtt tgtttctcct acactgcaac tccatgtttt cgaagtcccc 47760
   aaattcatag tatcattttt aaacctctac catcaccggg tgagagaagt gcataactca 47820
   tatgtatggc agtttaactg gactttctct tgtttccagt ttggggctat aaaggtttgt 47880
   aacaggtcct agtgtctggc agtgtgtgtt ctccagattt attatctttc ttcaagattg 47940
   gtttggctac tcttaggtgc ttatatttcc aaataatttt taaaggtatt agtttgtcaa 48000
   tttcccaaaa ccttgggctg gaatttctgg cagggtgaca ctaaatttat aggctagttt 48060
   ggaaagaact gaatcttgac acgttgaggc tttccattcc tgaatataat tatgcttcca 48120
   atttgtttgg ggtttctttt atttaaccag gaatgttgtg aatttgttgt catggctttc 48180
   gagtctttgg ttttccctag ataattaata tttttgttgt agaacataaa tagtttttat 48240
   cattctgatg atgttaatct gtcaactttg ctaaatttac tagtcactat tcgtaattta 48300
   tttctggatt cattgtaatt tctgtgtata ttatactgta tctgagttaa tattgtttta 48360
   tttcttattt tccatttctc atgggcttaa tgtctcttta tcacattcat tattgcatta 48420
   gctagaattt ctaggagagc attgaataga attggtgaca gtggggatcc ttgtttctca 48480
   tttctaatct gcaggaagca gtggaagttt tccatttcaa tattgagaat gatgcttgaa 48540
   gtagattttg gtagatattt tttatcagat tagagaagtt tgctgtcata tatatatatg 48600
   tcataattgt gtgtaaaatc ttgtcaaatc aacactctgc atctatttat ataattgtgt 48660
   gttagtccat ttccattgct ataaaggaat atctttgact gggtaattta taaataaaag 48720
   agtttcattt tggctcatgg ctctgcaggc tgtgcaagag gcataatgtg ggcaggcatc 48780
   tactcctagt aaggacttca ggtagctttc aatcttcgga gaaggtgaag gggaagcaag 48840
   catgtcacat ggcaagagag ggagtaagag aaaaaggagt agaagtgact ttaaacaacc 48900
   agctagtgtg tgaactaaca gaatatgaac tcacttatta tcatgggaag aggtttaagc 48960
   cactcatgaa gaattcaccc ccatgtccta aatacttctc acaccagaac ccacctccaa 49020
   cattgagaat cacatttcaa catgagattt ggaggggaca aatatccaca caacatcaga 49080
   ttatatatat tgggcttaca tttctcacta aatagaaata agtatgcaca tagagatgaa 49140
   ctggccttct tcatattgtt taggacttca gtgtttacat tcacaagaga tttggtttat 49200
   agttttattt ctcagaattt tcaagatttt tgatattaag attatgccag ccttataaaa 49260
   ctatgcaaga aatgtttcct aatgtgagac tgtttttatt gccgttccct ctccccactt 49320
   ttcttcttgt gatcacttag ttgtatcttt gaaatgcctg ataacttttg gttaaatacc 49380
   agatggtata tgtaagaaat tgcagaattg cagaggaggt ttatattctt caaagaaaga 49440
   ttcattcctc tcccatcggc agtcataatg acatgatacc tgttgtcatg attcagaatt 49500
   ggacataatg tgaatccagt tgaaaattga gtgtcagtgt tagcttagct ctctgaggtt 49560
   ctcctacctt tggaagcttg agaccaattt ttgtcttgac agctttgcag tgccttctat 49620
   cacatggttt ctgtatttat ccagacactc catttgccat ctcaaaccta ctcttttcca 49680
   atcaaaacca atatatatta ttagtaaaat aaaaaataat ataactgaaa gttgtatttt 49740
   caaggggaaa aagcaatgag acttggtaag gttgcaatta ctaatgtcat cttttgaaga 49800
   agacagaatt gataatttac ttagagatta tggggcttac cctagtggtc actgagttag 49860
   tacaagagaa aagacttcaa tgcactctgt tttcacaaac acaggagata accaggagac 49920
   tttcgcttct caaaattagg gtctgagggt aatggtaatg aaacttactt cttctcctat 49980
   aactaattaa atgagaaata tactgaagaa tcaggcacga gttgccagtc tccccagttc 50040
   actgcatcag ctttggtgac ctcctggagg ggaacgctgc atctgatgag ctcatgctgt 50100
   gcctgatgtc ttttgagccc tgagaagcca gagcccaagg ggattaccca ttgaatccct 50160
   tcttcgtgga atagcccagt gcagaaaaaa gcagcactgt gtggccaatt ctaccctctt 50220
   tacaaatcag atacatgtgt tcttctgcta caggctggat ccacttatct agctggagat 50280
   gatagtctga gacaggagtt ggcaaactgt ctctgtaatg ggccagatag taaatatttt 50340
   agacttttca ggcaatgtgg tttctgttgc aattcttgac atctgctatt gtagcatgat 50400
   agcagtcaca aacaatctgc aaatgaatga ggattgatat gttccaattt ttatagaaac 50460
   aaatgtaaat ttcctataaa atgtttgttt caaaaatatt gtttcttgtt tgcatttgtt 50520
   caatgtaagt acttttagct cacatactat acaaaaacag gtggcagggt ggacttgtgc 50580
   agggagtatt ttttattgat gcctaatata agacatccct gtccagtaga attttctgca 50640
   aagatgtgcg attgatttgt gcttttatgt catatttaaa gaatatttgt cagcacaaga 50700
   ttccaaagac tggctttcaa ctttcccaga atttttttgt tttagctctt atatctagat 50760
   caatgacata tttcaaatta atttttattt cataatatga agtaatagtg gtcatgcttc 50820
   aatgtctata aatttctact tattcaagta acatttgttg aaataaatct gctttggtat 50880
   caataacatt ttaatatagt acttgtttat aaatattcaa gggaatattt ttaaaaaaca 50940
   aaattataga ctatttatat gaaaatttag ggggtagaat ttttcctaac atttttatta 51000
   taaaattttt taaaataaat aacattacac acacatatgt atatgtacat atggcagtgt 51060
   gtatacatat ctactctgat atctgtccat ccatctattt acacctcaat acttcagtat 51120
   gtatagcaca ataacaatgg ttttctccta cataataata gcattcatac tgtggagtta 51180
   ttaatacaat aatattattt actatacagc ttttatttaa aatttcccaa ctttaaaata 51240
   atattttaat cacttttttc ttgcaattca ctctccagtc aagaaataag cacatagttt 51300
   cagtatcctg ttactttcca tctcctgtaa tcaagaagag tccatttcct ctgtttcctt 51360
   tcagtatgtt gatctctgtg aagagttgag caattagtca tttaaatttt cctggggctc 51420
   gttttgggta attgttttct aaatattata ttccagttat gactttggca agaaaactac 51480
   atagtatttt gaatattctg ctgaatcaca gtggtgaggc aggggcacat aatgtcacat 51540
   atgccattat tggttatgct aagatcctca cctaataagt tggaatctgc cagatctttc 51600
   cattgaaggg gttctgcttt ttatgcgtga atgatgaaaa tctatggcat ggtacttcaa 51660
   ggcagaacac cctgtttccc aagtcacaca tccaatagtt ttatctttca ttaatgatcc 51720
   tcatctgacc tgatgatctt gacctgaatg gttacaaata ggtgattttt ctaatttcat 51780
   cattccttcc gtatttttaa gctaggatta atctgtaatg aatatctctc ccatatcttt 51840
   aaatgaagac aaacaaactg catggcaatg acaagaactc aatagtttta tccttgccac 51900
   tacacttgtt accatcccag aagatgtcat ttgaaattca atcatatgtt tattcacaaa 51960
   tgccctgtga acagatactg tggccatttc ccatgtcaac agagacttca acttttagtt 52020
   atactctaca cataaggttg gtgaaaactg aagtgatgga atagagaagc aagtgtggac 52080
   agccactagt ttccaagctt gatgaaaagg aggacagatt cagaaggttg catccaagta 52140
   tccaagtact caggacttca aatgtgattg cagggcactt tagcaagatt attgtcatgg 52200
   gccttaagtt catgactctt attacttggt ctatccatct ggaaatggta ctgcccttct 52260
   ttggaacggg atttcctcat ctgcacatca aaagatttaa ctacatgatt accactgttt 52320
   cttcaacctt catggcttct ttacagctca gttcatctat gtctcttgtt tctagggcac 52380
   aaccatatta atttccctga cttctgtgct acatgacaac aaagaatttc ccaacccaga 52440
   gatgtttgac cctcatcact ttctggatga aggtggcaat tttaagaaaa gtaaatactt 52500
   catgcctttc tcagcaggta atataaattt atttccattt gtgtttcagg gtacaagata 52560
   acttttttga tccattggaa cttacatgtg cctcctctgc agtggtacaa ttactctttg 52620
   tacatgatca agagcactgt tctgaatgcc tgtgtacacc ctgctcatga tacatcctaa 52680
   ttattgggcc agattagtgg actttgggga gttaatccaa ttcttccaaa ttgagaaagc 52740
   tgaaatatag gttggttgaa ttctgcctct aggtacacca gtgaggtacc caagaactcc 52800
   tcctggaaga taaaactaat tacattttcc tcactagcca tgaggaagtt atctcactcc 52860
   agaacttcac tgagtgtctt ccacatggtg tccctcaccc cctaggctgg gcttgtagga 52920
   taaaattatc cacaaacaca gaatagggtc ttaaaaggct cacttctgag tttggaaagc 52980
   agagtaaaca gatcattgta gttcaatagg actgaggctg tgatatggaa agaacaggct 53040
   gttgggggtt gggaggtaga cggaaaagct gtctgcttct tgtactctta taccccaaag 53100
   tgaggcataa gtaatatttt aatagcagta aagacatttg agctacctca aaggaggcag 53160
   agaggatgaa aagaagagaa gacagggcta ttaaaggaga taatgagcca caggagcagg 53220
   acattggctc taaataaagg atattagaac ctttgccaaa tgaccatgga tgggatgagg 53280
   gggacattgg gaatgtagca ggatactctg cagtgatgca gagcaccatg ctgttcccct 53340
   agtcatggcc atgtatattg gatgttggat ctccatactt gaaatgtgtg tgctgagcat 53400
   ctggtgatag aatctccttc ctttattcta tgacctttaa tgtctgcttt atatctgcca 53460
   ctgtagatac caagacaaca agaaaagaaa ccttccttca agcattcaca tttagcacat 53520
   gtattagtcc actcttacat tgccttaaag aactacctga gattgggtaa tttatgaaga 53580
   aaagaggttt aattcactca cagatccaca ggctgtacag gaagcatggc tgggagacct 53640
   caagaaactt gcaatcatgg tgggaggcag agcggaagca agcacatctt tccatggcag 53700
   agcagaagag agagaacaat gaggaaaggc tacatacctt taaacaacca gatctcatga 53760
   gaactcactc actatcatga gaacagaaag ggggaaatta tcccccatga ttcaatcacc 53820
   tctcaccagg tatctccccc aacattggga attacagttt gacatgagat ttgagtaagg 53880
   acacagagcc caaccatatc gcacactaat aaataaataa ataaatcagt aattattatt 53940
   ctaggtgatc catgctgtct gtttcttctg ccataacaaa atatccaaga ctaggcaatt 54000
   gataaataat ggaaatttat ttctcacagt tctgaaggct gggagttcca agatcgatgt 54060
   gtcagcaagt tcagtgtctg atgtgggcct gttccttaca gataatgccc cctctgtatc 54120
   cttatatggc agaagggcaa aaaggcaaaa ggggagaaat ggctcccttg catctcttga 54180
   tgttattatt ttagcaagga cagagccctc ctgacttatt cacttcctaa aaggagccat 54240
   ctctttagta atgttgcatt gggattatgt gtcaacatat gaaattgggg gaggcatatt 54300
   cagaccatag cacatttttc aatggaaata taatgttgag gaaacccaga gaaggcaaca 54360
   ttttcttgct caaggagatg agaaagaggg taaaaaagga gataaaattt gacctatgtc 54420
   ctgactgtgg taatagaaaa gttcatcttg gctaaaagga gcagcatgat ataaaatttg 54480
   aaacctcatg gtgtgttgga gactgatgat gagtggctat gcctagagtt gacagtatcg 54540
   gatttgaaga gtgtaaggag tgatgtggat catcagactg gaaacagaat gtgagggtcc 54600
   agatcaatcc attgggacct tatcctatag gacatacagg gaagccattt aaagttttaa 54660
   agtgagaagg tgacatgttt agacatgtgc tcctgaaagt acctagagga aaaaaatctt 54720
   tggctgcata ttgagccaga aatacaaagg gaaatacagt atgttagcct cctcctctaa 54780
   gcccttctca gttcaaccca ctggacaaga aatgtatgtt tctaaagaaa gattgatgaa 54840
   gacatttaaa gtctcttgaa agattttaat aaagtgcttg gcatgtagct ggtactcaac 54900
   aaatatttgt tgaatacagg gtgcctgtta agatctgata ttaggtgaag agtaagtatg 54960
   tccattcatt tttcagttgc ctatacatcc atccattcat ccatttatcc atccactcat 55020
   ccatccattc attcatgcat gcacccatcc acccatctat ctcttcatct cttctacgat 55080
   acactgaaca gttattgcat attctgtttg tgccagttac agagacagtg tttgtcactg 55140
   tcacagttac gcatgaggag taactgctct ctgtgtttgc tattttcagg aaaacggatt 55200
   tgtgtgggag aagccctggc cggcatggag ctgtttttat tcctgacctc cattttacag 55260
   aactttaacc tgaaatctct ggttgaccca aagaaccttg acaccactcc agttgtcaat 55320
   ggatttgcct ctgtgccgcc cttctaccag ctgtgcttca ttcctgtctg aagaagagca 55380
   gatggcctgg ctgctgctgt gcagtccctg cagctctctt tcctctgggg cattatccat 55440
   ctttcactat ctgtaatgcc ttttctcacc tgtcatctca cattttccct tccctgaaga 55500
   tctagtgaac attcgacctc cattacggag agtttcctat gtttcactgt gcaaatatat 55560
   ctgctattct ccatactctg taacagttgc attgactgtc acataatgct catacttatc 55620
   taatgttgag ttattaatat gttattatta aatagagaaa tatgatttgt gtattataat 55680
   tcaaaggcat ttcttttctg catgttctaa ataaaaagca ttattatttg ctgagtcagt 55740
   ttattagacc ttccttcttt tatgcataat gtaggtcaga aattaaagaa aatagagttc 55800
   caggaggcca tgctggttct caaaatgata aggacagaaa ggacaaagag gaagagggta 55860
   gggaagctat tttgggtgag tgttagagtt acttgaggat tggatttgaa agtgagaaac 55920
   tgtgtccagg ggcagctcta acctctaggg aaatattcag aggatcagtc aaagggtgga 55980
   atggacatta aatgctagaa ttcttatatc cacattggtg ttcctttttt tttgagacaa 56040
   agtcttgctc tgtcacccag gctggagtgc agtggtgtga tctcagctct ctataacctc 56100
   cgcctcccag gttcaagtga ttctcctgcc tcagcctcct gagtagctgg gattacaggt 56160
   gcatgccacc acacctggct aattttttgt atttttagta cagacgggtt ttcaccgtgt 56220
   tagccaggat ggtcttaatc tcctgacctt gtgatctgcc tgcctcagcc tcccaaagtg 56280
   ctgggattac aggtgtgagc cactgcgcct ggcctggtgt tactttgaag tgtcattact 56340
   ttatctctaa ataaagaatc agattacttt tattacttca tgtttccaac ttagaatgat 56400
   gtaatgaagt ataaatacat gctttcatat cgctgctcat gtgttctgtc atgtttctct 56460
   cttcaaacat aaacaaaatt tctttttggt ttttgtttgt ttgtttgttt gttttgagat 56520
   gtagtctcgc ttttgttgcc caggctggag tacaatggca tgatcttggc tcactgcaat 56580
   ctccacctcc agggttcaag tgattctcct gcttcagcct cccaagtagc tgggattaca 56640
   ggtgccaacc cccataccag gctaattttt gtatttttag tagagacagc atttcaccat 56700
   gctggccaga ctagtcttga actcctgacc tcaagtgatc cacccatctc ggactcccaa 56760
   agtgctggga ttacaggcat gagctaccat gcttggctgg gcaaaatttc taagaagaat 56820
   tacaaagatt taatctctct cagcttctcc tacattattt tacttttaac ataagtggga 56880
   tgataatttt atgctatggt cctaaatata attagcctca tgtcatctcc aaagaataga 56940
   caactaagta tcttatgtta aattatattc accacttttc ctgtcttcta cttgaaatgt 57000
   aagaaataag aagtaacata atttgttatg cttctcacag ctcatctccc agaagaagtt 57060
   acttacattt tggaatacgt tttaaagata atatgtggat acaggtgtac atgaacatct 57120
   atatatgtct gtgtacatta tgtgtattta cccattttat atatatgcat atatatatat 57180
   atatacacac acatatatgt aacttatgca cacatacata tagggttaaa tgtttattac 57240
   aatattgggc tcatatcaca catcttgtat taaaacttgt tattttccta tcaagtaata 57300
   tggcagcaaa acctctaagg tgatagaaac atagggaaat gattgagaga gataatagaa 57360
   gagaaagtgg aaatgagtat tacattaatg ggaagatcca aatggaaatt atacattata 57420
   cctttcaacc ttcttcattg ggaaatgcaa atgtattaat gtagtgttag tagaggtgct 57480
   ggcaaacacc cctaggtgag ataaatcaag tagatgtcaa gtctgttttc tcctagagac 57540
   tgtagtgaat aatctgtttt cctgcctatt tcacctccta aaggctgcct gcattccttg 57600
   gctcatgaac tcttccttct ttcactccaa acacttgctt ctgttgtcac atctcctact 57660
   cctactactg actcttattc tcacatctcc cttacattag aaatgcaaaa tgctttttcc 57720
   ctggtgtcac aaag 57734

## Description

The present invention relates to methods, kits and devices for assaying for warfarin sensitivity in a patient. In particular, it relates to an assay that can be used in a microfluidic device and which can provide a sufficiently rapid and robust result to allow for point of care diagnosis. The invention utilises pairs of oligonucleotide primers specific for genomic regions flanking *CYP2C9^{∗}2, CYP2C9^{∗}3* and *VKORC1* single nucleotide polymorphisms (SNPs) to amplify (in a multiplexed reaction) the regions of interest; and, in certain embodiments, the invention also uses specific probes for capturing the amplified regions to allow for analysis. Advantageously the capture probes allow discrimination between different alleles using allele specific probes that also allow us to detect heterozygotes as well as homozygotes and allows for identification of one or more SNPs associated with warfarin sensitivity in the clinical sample.

Clotting (thickening) is a complex process, involving a number of clotting factors produced by the liver, which helps control bleeding. The clotting factors work with cells that trigger the clotting process (platelets) to ensure blood clots, or coagulates, effectively. The liver requires a good supply of vitamin K for certain clotting factors to be produced. Warfarin is the most widely prescribed oral anticlotting/ anticoagulant. Warfarin blocks one of the enzymes that uses vitamin K to produce clotting factors, and this in turn disrupts the clotting process, resulting in it taking longer for blood to clot.

Warfarin at appropriate levels is often prescribed for its antithrombotic benefits to prevent blood clots in people with heart disease, people who have replacement heart valves, people with an irregular heart beat (atrial fibrillation), or those with a history of heart attack, stroke, or a prior blood clot in the deep veins of the arms or legs (deep vein thrombosis (DVT)). However, appropriate dosage levels change from individual to individual and not everyone metabolises warfarin the same way. Individuals with warfarin sensitivity take longer than normal to metabolise warfarin, so the medication is in their body longer than usual and they typically require lower doses. These individuals are classified as "slow metabolizers" of warfarin. Other people with warfarin sensitivity do not need as much drug to prevent clots because their clot forming process is already slower than average and can be inhibited by low warfarin doses. If people with warfarin sensitivity take the average dose (or more) of warfarin, they are at risk of an overdose, which can cause abnormal bleeding in the brain, gastrointestinal tract, or other tissues, and may lead to serious health problems or death.

The antithrombotic benefits of warfarin are therefore countered by a narrow therapeutic index that contributes to excessive bleeding or cerebrovascular clotting and stroke in some patients.

A relationship between allelic variants and warfarin sensitivity has been identified, with studies showing considerable allelic variability in CYP2C9 (genbank accession no NG_008385, SEQ ID No 15) and VKORC1 (genbank accession no NG_011564, SEQ ID No 14). Certain alleles are associated with increased sensitivity to warfarin. Several variant *CYP2C9* alleles are associated with reduced enzyme activity and lower clearance rates of warfarin. Patients who carry at least one copy of such a variant allele (such as *CYP2C9^{∗}2* (rs1799853) and *CYP2C9^{∗}3 (rs1057910))* have reduced metabolism leading to higher warfarin concentrations. *CYP2C9^{∗}2* allele contains a C-to-T transition, leading to substitution of cysteine by arginine at amino acid position 144. The *CYP2C9^{∗}3* allele is defined by an A-to-C nucleotide substitution that leads to an exchange of leucine by isoleucine at amino acid position 359. On average, they require a lower daily warfarin dose than patients who are homozygous for the wild-type *CYP2C9^{∗}1* allele.

The *VKORC1* gene encodes the vitamin K epoxide reductase enzyme, the target of warfarin. Patients who carry the -1639G>A polymorphism (rs9923231) in the promoter region of the *VKORC1* gene are more sensitive to warfarin and require lower doses.

Detection of mutations is typically carried out by direct sequencing of samples. This can be a fairly lengthy and laborious process and requires a certain level of knowledge and experience. It would therefore be beneficial to provide methods and/or kits or devices to allow for rapid personalised indications of warfarin sensitivity.

Assays for assessing warfarin sensitivity are known in the prior art. A lateral flow cell for warfarin genotyping from saliva is described by Thomas et al., (2014). Lyon et al. (2008) describe a number of commercially available assays for determining warfarin sensitivity. Sheth (2015) describes a point-of-care device utilising silicone nanowires for the genotyping of CYP2C9 and VKORC1 alleles. The present invention aims to mitigate the problems associated with the prior art. Reference to a multiplex reaction or multiplexing relates to the simultaneous measurement or detection of multiple amplicons in a single run/cycle of the assay or the simultaneous amplification of multiple different amplicons.

In the document, references to *^{∗}*2 refer to CYP2C9*2(rs1799853), references to ^{∗}3 refer to *CYP2C9^{∗}3 (rs1057910)* and references to VK refer to *VKORC1-*1639G>A (rs9923231).

The -1639G>A transition of rs9923231 relates to position 3588 of NG_011564 (SEQ ID No 14). The C>T transition of rs1799853 relates to position 8633 of NG_008385 (SEQ ID No 15). The A>C transition of rs1057910 relates to position 47639 of NG_008385 (SEQ ID No 15).

According to the present invention there is provided a rapid method Z , as defined in claim 1, of determining
warfarin sensitivity comprising; contacting a sample with pairs of oligonucleotide primers, each pair comprising a forward primer and a reverse primer, that are operable to bind genomic regions flanking CYP2C9*2(rs1799853), *CYP2C9^{∗}3(rs1057910)* and *VKORC1-*1639G>A single nucleotide polymorphism (SNP) sites and to amplify genomic regions to give amplicons comprising said SNP sites;
amplifying the SNP sites by polymerase chain reaction by annealing the primer pairs to the nucleic acid in the sample; simultaneously extending the annealed primers to synthesize an extension product, wherein each extension product after separation from the nucleic acid, serves as a template for the synthesis of an extension product for the other primer of each pair; and
detecting the amplified products, if present, to indicate the presence or absence of a single nucleotide polymorphism (SNP) associated with warfarin sensitivity in the sample, wherein detection of single nucleotide polymorphisms is indicative of warfarin sensitivity.

Preferably the SNP sites are amplified substantially simultaneously as a multiplexed reaction. This allows for greater speed but such multiplexed reactions can be challenging due to non-specific binding between primers or primers interfering with each other. This allows a result to be achieved in a microfluidics system in a relatively short timeframe such that it is appropriate for a POC diagnosis.

Z The primer pairs are as defined in the claims and Z said primer pairs are each selected to give an amplicon of less than 150 bases

Advantageously the inventors have found that by utilising reduced length PCR products/amplicons (amplicons of less than 150 bases) the time required for the amplification process is rapid. Typically, amplicons for this type of reaction are longer as it is easier to select appropriate primers if the sequence to be amplified is extended outwards to give more sequence options for primer design, this is particularly the case when the primers are for use in multiplex reactions.

Preferably at least some of the PCR reagents are provided in excess compared to standard PCR protocols, where standard or typical PCR reagent concentrations are;

| **Standard PCR Component** | **Standard Final Concentration** |
|---|---|
| 10X Standard *Taq* Reaction Buffer | 1X |
| 10 mM dNTPs | 200 µM |
| 10 µM Forward Primer | 0.2 µM (0.05-1 µM) |
| 10 µM Reverse Primer | 0.2 µM (0.05-1 µM) |
| Template DNA | <1,000 ng |
| *Taq* DNA Polymerase | 1.25 units/50 µl PCR |
| Nuclease-free water | to 50µl final volume |

This is to maximise the reaction kinetics and is particularly suited to microfluidic environment where there are challenges with standard PCR reactions such as the adsorption of reagents to the large surface areas. For example, it is fairly typical for standard PCR protocols for the primer concentration to be 0.5µM, whereas the present invention has primer concentrations where at least one of the primers has a concentration of at least 3, more preferably, 4, times that amount i.e. 1.5µM and above, and can be up to 12 times or more in some instances. Optionally it is the primers that are in excess. Optionally they may be more than 3-fold the amount used in standard PCR protocols.

The polymerase may also be at a higher concentration than is standard for a PCR reaction. For example, the polymerase may form 2% or more of the reaction mix.

Z The pairs of oligonucleotide primers are as defined in claim 1.

Preferably the pairs of oligonucleotide primers are;

| **Primer name** | **Sequence** | **Product/amplicon size (bp)** | **SEQ ID** |
|---|---|---|---|
| ^{∗}2_v1_F | GGCGTTTCTCCCTCATGACGC | 126 | SEQ ID No 1 |
| ^{∗}2_v1_R | GAGTAGGGTCACCCACCCTTGG | | SEQ ID No 2 |
| ^{∗}3_v1_F | GCAAGACAGGAGCCACATGCC | 112 | SEQ ID No 3 |
| ^{∗}3_v2_R | GAATTTAATGTCACAGGTCACTGC | | SEQ ID No 4 |
| VK_v1_F | GGATTATAGGCGTGAGCCACC | 97 | SEQ ID No 5 |
| VK_v1_R | GGTAGGTGCAACAGTAAGGGATCC | | SEQ ID No 6 |

In the table above the F and R notations in the primer name indicate forward or reverse primers. Notably, it is generally accepted that the optimal length of PCR primers is 18-22bp or greater, however, some of the ones we use are 16bp in length.

Alternatively, the primer ^{∗}3_v2_R primer is a ^{∗}3_v2_R' primer with a sequence of CTGAATTTAATGTCACAGGTCACTGCATG.

Alternatively, the primers have at least 80% sequence homology to the above primers. Alternatively, the primers have at least 90% sequence homology to the above primers. Alternatively, the primers have at least 95% sequence homology to the above primers.

Preferably the PCR reaction takes less than 10 minutes, more preferably less than 9 minutes, more preferably less than 8 minutes. Advantageously, in the present invention this rapid reaction can occur in a microfluidic environment due to the selection of shorter length amplicons, and the appropriate use of higher than standard concentrations of PCR reagents. This allows the generation of sufficient amplicon to allow for hybridisation to occur in a sufficiently short period of time, making it suitable for point of care (POC) testing where rapid results are important.

Preferably the respective primer concentrations CYP2C9^{∗}2:CYP2C9^{∗}3:VKORC1 are 1:2:4 or 1:2:6. For example the CYP2C9^{∗}2:CYP2C9^{∗}3:VKORC1 final primer concentrations may be 1µM;2µM;4µM or 0.5µM;1µM;2µM or 1µM:2µM:6µM. Alternatively, when the ^{∗}3_v2_R' primer is used the concentrations are:

| | Final concentration (µM) |
|---|---|
| ^{∗}2F | 2.5 |
| ^{∗}2R | 2.5 |
| ^{∗}3F | 2 |
| ^{∗}3R' | 5 |
| vkF | 5 |
| vkR | 2 |

These primer concentrations and ratios were used to give a better hybridisation signal for all products. Previously, when the ^{∗}3_v2_R' primer was used they were optimised to give even gel bands when assessed by gel electrophoresis.

These concentrations have been used for hybridisations from buccal samples and results have been verified using a Agena MassArray^{™}

Advantageously, by using the relative primer concentrations identified by the inventors, this reduces non-specific amplification and also achieves relatively equal amplification of the three PCR products/amplicons.

Preferably for each primer pair, one of the pair is labelled. Most preferably this is a fluorescent label or a charged mass tag. Alternatively, labels may be a dye, a radioactive isotope, a chemiluminescent label, a fluorescent moiety, a bioluminescent label an enzyme, and combinations thereof

Preferably for the fluorescently labelled *CYP2C9^{∗}2* primer pair the labelled primer is in excess of the unlabelled primer. Alternatively, the labelled ^{∗}2 and unlabelled ^{∗}2 are in equal amounts.

Preferably for the CYP2C9^{∗}3 primer pair the labelled primer is in excess of the unlabelled primer.

Preferably for the VKORC1 primer pair the labelled primer is in excess of the unlabelled primer.

The excess amount of labelled primer is most relevant in the *CYP2C9^{∗}3* primer pair and the VKORC1 primer pair.

Optionally the method further includes providing a plurality of capture probes capable of selectively binding to either wild type or mutant versions of CYP2C9*2(rs1799853),

*CYP2C9^{∗}3(rs1057910)* and *VKORC1-639G>A*,wherein the binding of amplified products to capture probes indicates the presence or absence of a SNP associated with warfarin sensitivity in the clinical sample.

Advantageously the capture probes allow discrimination between different alleles using allele specific probes that also allow us to detect heterozygotes as well as homozygotes. The capture probes can be integrated onto a microarray. The microarray and capture probes can be used in conjunction with the output of the PCR to determine the genotype for each of *CYP2C9*2(rs1799853), CYP2C^{∗}3(rs1057910)* and *VKORC1-1639G>A.*

It is possible that the PCR can be used independently of the microarray and capture probes. Identification or detection can then follow by any appropriate means. Optionally the probes are;

| **Probe name** | **Sequence** |
|---|---|
| ^{∗}2-WT probe | GAA CAC GGT CCT CAA TGC T |
| ^{∗}2-M probe | GAA CAC AGT CCT CAA TGC T |
| ^{∗}3-WT probe | ACG AGG TCC AGA GAT ACA TTG AC |
| ^{∗}3-M probe | CGA GGT CCA GAG ATA CCT TGA C |
| VK-WT probe | CAA CCA TTG GCC GGG T |
| VK-M probe | CAA CCA TTG GCC AGG T |

In the table above the WT and M notations in the probe name indicate Wild-Type or Mutant probes respectively.

Alternatively, the probes have at least 80% sequence homology to the above probes. Alternatively, the probes have at least 90% sequence homology to the above probes. Alternatively, the probes have at least 95% sequence homology to the above probes. Preferably the probes can bind under stringent hybridisation conditions.

Optionally the capture probes are bound to sensitive detection devices.

Optionally the sensitive detection devices are field effect transistors (FETs). Optionally the sensitive detection devices are nanowires.

Optionally the capture probes comprise a detectable label. Optionally this is a fluorescent label. Alternatively, it is a mass charge. Alternatively, labels may be, a dye, a radioactive isotope, a chemiluminescent label, a fluorescent moiety, a bioluminescent label an enzyme, and combinations thereof

Optionally the sample is a whole blood sample. Optionally the sample is buccal cells. Optionally the sample is whole blood, plasma, serum, CSF, feces, urine, cultured cells, saliva, cervical or urethral swab, sputum and other biological fluids.

Optionally, the PCR reaction uses a Phire^{™} DNA polymerase. Phire^{™} Hot Start II DNA Polymerase incorporates a dsDNA-binding domain that allows short extension times (10-15 s/kb), helps improve yields, and can increase fidelity two-fold compared to Taq DNA polymerase. Alternatively, any DNA polymerase with the ability to achieve less than 10 minute PCR could be used.

According to an aspect of the present invention there is provided a kit for rapid determination of warfarin sensitivity, comprising pairs of oligonucleotide primers, each pair comprising a forward primer and a reverse primer, that are operable to bind genomic regions flanking CYP2C9*2(rs1799853), *CYP2C9^{∗}3(rs1057910)* and *VKORC1-*1639G>A single nucleotide polymorphism (SNP) sites and to amplify genomic regions to give amplicons comprising said SNP sites. The primer pairs are as defined in the claims.

Said primer pairs are each selected to give an amplicon of less than 150 bases

Preferably the pairs of oligonucleotide primers are;

| **Primer name** | **Sequence** | **Product/amplicon size (bp)** |
|---|---|---|
| ^{∗}2_v1_F | GGCGTTTCTCCCTCATGACGC | 126 |
| ^{∗}2_v1_R | GAGTAGGGTCACCCACCCTTGG | |
| ^{∗}3_v1_F | GCAAGACAGGAGCCACATGCC | 112 |
| ^{∗}3_v2_R | GAATTTAATGTCACAGGTCACTGC | |
| VK_v1_F | GGATTATAGGCGTGAGCCACC | 97 |
| VK_v1_R | GGTAGGTGCAACAGTAAGGGATCC | |

In the table above the F and R notations in the primer name indicate forward or reverse primers.

Alternatively, the primer ^{∗}3_v2_R primer is a ^{∗}3_v2_R' primer with a sequence of CTGAATTTAATGTCACAGGTCACTGCATG.

Alternatively, the primers have at least 80% sequence homology to the above primers.

Alternatively, the primers have at least 90% sequence homology to the above primers.

Alternatively, the primers have at least 95% sequence homology to the above primers.

Optionally the kit further includes a plurality of capture probes capable of selectively binding to either wild type or mutant versions of CYP2C9*2(rs1799853), *CYP2C9^{∗}3(rs1057910)* and *VKORC1-1639G>A .*

Optionally the probes are;

| **Probe name** | **Sequence** |
|---|---|
| ^{∗}2-WT probe | GAA CAC GGT CCT CAA TGC T |
| ^{∗}2-M probe | GAA CAC AGT CCT CAA TGC T |
| ^{∗}3-WT probe | ACG AGG TCC AGA GAT ACA TTG AC |
| ^{∗}3-M probe | CGA GGT CCA GAG ATA CCT TGA C |
| VK-WT probe | CAA CCA TTG GCC GGG T |
| VK-M probe | CAA CCA TTG GCC AGG T |

Alternatively, the probes have at least 80% sequence homology to the above probes.

Alternatively, the probes have at least 90% sequence homology to the above probes.

Alternatively, the probes have at least 95% sequence homology to the above probes.

Preferably the probes can bind under stringent hybridisation conditions.

Optionally the capture probes are bound to sensitive detection devices.

Optionally the sensitive detection devices are nanowires.

According to an aspect of the present invention there is provided a device , as defined in claim 13, for rapid
determination of warfarin sensitivity, comprising;
a sample reception port;
an amplification region for amplifying genomic regions to give amplicons comprising CYP2C9*2(rs1799853), *CYP2C9^{∗}3(rs1057910)* and *VKORC1-1639G>A* single nucleotide polymorphism (SNP) sites, said region in use containing pairs of oligonucleotide primers, each pair comprising a forward primer and a reverse primer, that are operable to bind genomic regions flanking CYP2C9*2(rs1799853),
*CYP2C9^{∗}3(rs1057910)* and *VKORC1*-*1639G>A* single nucleotide polymorphism (SNP) sites;
a detection region, said detection region being downstream of the amplification region, for detecting the amplicons to indicate the presence or absence of a single nucleotide polymorphism (SNP) associated with warfarin sensitivity in the sample.

Preferably the pairs of oligonucleotide primers are;

| **Primer name** | **Sequence** | **Product/amplicon size (bp)** |
|---|---|---|
| ^{∗}2_v1_F | GGCGTTTCTCCCTCATGACGC | 126 |
| ^{∗}2_v1_R | GAGTAGGGTCACCCACCCTTGG | |
| ^{∗}3_v1_F | GCAAGACAGGAGCCACATGCC | 112 |
| ^{∗}3_v2_R | GAATTTAATGTCACAGGTCACTGC | |
| VK_v1_F | GGATTATAGGCGTGAGCCACC | 97 |
| VK_v1_R | GGTAGGTGCAACAGTAAGGGATCC | |

In the table above the F and R notations in the primer name indicate forward or reverse primers.

Alternatively, the primer ^{∗}3_v2_R primer is a ^{∗}3_v2_R' primer with a sequence of CTGAATTTAATGTCACAGGTCACTGCATG.

Alternatively, the primers have at least 80% sequence homology to the above primers.

Alternatively, the primers have at least 90% sequence homology to the above primers.

Alternatively, the primers have at least 95% sequence homology to the above primers.

Preferably the detection region comprises a plurality of capture probes able to capture one or more amplified products.

Optionally the probes are;

| **Probe name** | **Sequence** |
|---|---|
| ^{∗}2-WT probe | GAA CAC GGT CCT CAA TGC T |
| ^{∗}2-M probe | GAA CAC AGT CCT CAA TGC T |
| ^{∗}3-WT probe | ACG AGG TCC AGA GAT ACA TTG AC |
| ^{∗}3-M probe | CGA GGT CCA GAG ATA CCT TGA C |
| VK-WT probe | CAA CCA TTG GCC GGG T |
| VK-M probe | CAA CCA TTG GCC AGG T |

Alternatively, the probes have at least 80% sequence homology to the above probes.

Alternatively, the probes have at least 90% sequence homology to the above probes.

Alternatively, the probes have at least 95% sequence homology to the above probes.

Preferably the probes can bind under stringent hybridisation conditions.

Optionally the detection region comprises sensitive detection devices.

Optionally the detection region comprises a field effect transistor (FET). Optionally this may be a nanowire FET. Preferably the capture probes are bound to the FET or nanowire FET such that a binding event with an amplicon results in a measureable change in charge density or other electrical property.

Preferably the device is a continuous flow device. The device may be in the form of a cartridge or cassette.

Preferably the device is part of a point of care device. Ideally, it is of an appropriate size to be handheld.

Alternatively, the detection region may comprise a surface to which capture probes are bound and a viewing window that allows the detection region to be viewed or imaged. Most preferably in this case the amplicons comprise fluorophores. and the means for detecting is a fluorescence imager.

Preferably the device comprises, or is associated with, a means for visually imaging bound amplicons. The means for visually imaging bound amplicons is a fluorescence reader.

Optionally the detection region comprises a plurality of capture probes capable of selectively binding to either wild type or mutant versions of C*YP2C9*^{∗}*2*(rsI799853), *CYP2C9^{∗}3(rs1057910)* and *VKORC1-1639G>A .*

In order to provide a better understanding of the present invention, embodiments will be descried by way of example only and with reference to the flowing figures in which;

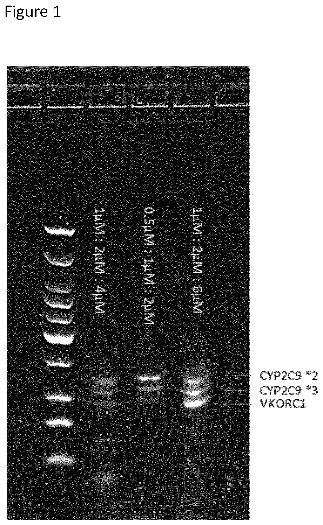

Figure 1 shows a gel image of the results of a continuous flow PCR reaction using Phire^{™} PCR mix and the primers of the invention. Different ratios of primers were used. Ratios in the image have the following format CYP2C9^{∗}2: CYP2C9^{∗}3: VK0RC1 final primer concentration; and

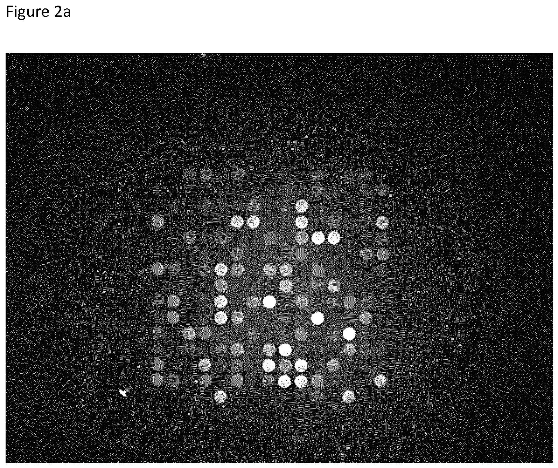

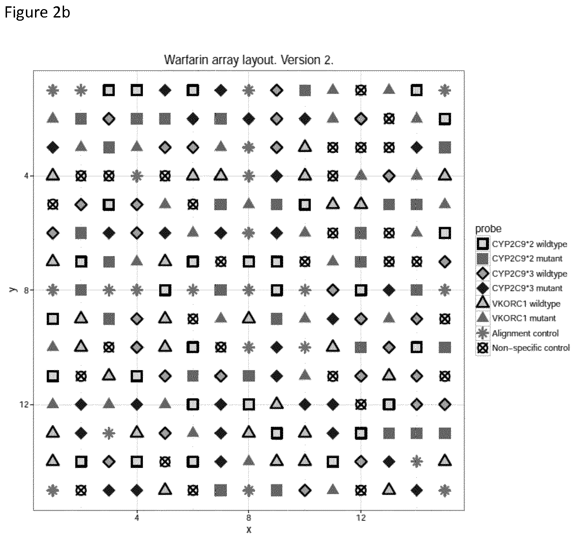

Figure 2a shows an imaged hybridisation of the multiplex PCR using an imaging rig, whilst Figure 2b shows the probe layout within the array itself. Control (alignment) probes are not visible in this image. To generate a genotype, each probe pair (both probes specific to each SNP) is relevant to one another and not to the other probe pairs. Therefore in Figure 2 the CYP2C9^{∗}2 Wildtype probes are visible whereas the mutant is not. The wildtype and mutant CYP2C9 ^{∗}3 probes are visible. The wildtype and mutant VKORC1 probes are visible.

### Exemplary assay

In one embodiment, a multiplexed PCR reaction can be performed on a slide using a blood sample from a patient to determine warfarin sensitivity.

### Sample

A sample of whole blood is obtained and is used at a 2% v/v ratio in the reaction.

### Primers

The following primers are Cy5 labelled at 5' end.

Only one in each of the pairs of primers is Cy5 labelled. The forward for ^{∗}2, the reverse for ^{∗}3 and the forward for VK.

| **Primer name** | **Sequence** | **Product/amplicon size (bp)** |
|---|---|---|
| ^{∗}2_v1_F | GGCGTTTCTCCCTCATGACGC | 126 |
| ^{∗}2_v1_R | GAGTAGGGTCACCCACCCTTGG | |
| ^{∗}3_v1_F | GCAAGACAGGAGCCACATGCC | 112 |
| ^{∗}3_v2_R | GAATTTAATGTCACAGGTCACTGC | |
| VK_v1_F | GGATTATAGGCGTGAGCCACC | 97 |
| VK_v1_R | GGTAGGTGCAACAGTAAGGGATCC | |

It would however be appreciated by one skilled in the art that alternative dyes could be used to label the primers. In some embodiments, a dual-fluorophore can be used to boost fluorescence signal and/or reduce time needed to reach detectable signal.

Primer concentrations for this embodiment are shown below;

| Primer | Final Concentration (µM) |
|---|---|
| ^{∗}2_v1_F | 0.33 |
| ^{∗}2_v1_R | 0.33 |
| ^{∗}3_v1_F | 0.25 |
| ^{∗}3_v2_R | 1.25 |
| VK_v1_F | 3.33 |
| VK_v1_R | 0.83 |

Notably, for the ^{∗}3 and VK primers higher concentrations of one primer in the primer pair. Asymmetry of primers aids downstream hybridisation by limiting single stranded complements that could compete for binding to the fluorescent strand

200uM of each dNTP is used, along with the Phire^{™} buffer. The Phire II^{™} polymerase is used at 2% (V/V). The Phire II^{™} polymerase is of particular use (although is not the only available polymerase) as it can be used with whole blood - one skilled in the art would appreciate that if the sample had been pre-processed e.g. to provide purified nucleic acid, then a broader range of polymerases would be available for use.

In an alternative embodiment, the ^{∗}3_v2_R primer is a ^{∗}3_v2_R' primer with a sequence of CTGAATTTAATGTCACAGGTCACTGCATG. When the ^{∗}3_v2_R' primer is used the concentrations can be:

| | Final concentration (µM) |
|---|---|
| ^{∗}2F | 2.5 |
| ^{∗}2R | 2.5 |
| ^{∗}3F | 2 |
| ^{∗}3R' | 5 |
| vkF | 5 |
| vkR | 2 |

Again, there is asymmetry in the concentrations of the ^{∗}3 and VK primers.

A polymerase chain reaction is then carried out. In this example, the PCR reaction is carried out on a benchtop PCR machine, however it will be appreciated that the PCR could be carried out on a chip, cassette or similar which may be integrated with upstream and/or downstream processing in the same device.

### PCR

The following benchtop PCR protocol is exemplary to provide amplicons for further detection and/or processing;

An example for a 120ul reaction (volumes can be adjusted if necessary depending on reaction volume)

| **PCR Constituent** | **Volume (ul)** | **Final concentration in Reaction** |
|---|---|---|
| Water | 81.2 | - |
| 5x Phire buffer | 24 | 1× |
| dNTPs (10mM each) | 2.4 | 200uM each |
| PS145 (100µM) | 0.4 | 0.333333333 µM |
| SN42 (100µM) | 0.4 | 0.333333333 µM |
| SN43 (100µM) | 0.3 | 0.25 µM |
| PS146 (100µM) | 1.5 | 1.25 µM |
| PS120 (100µM) | 4 | 3.333333333 µM |
| PS121 (100µM) | 1 | 0.833333333 µM |
| Blood | 2.4 | 2% |
| Phire Polymerase | 2.4 | 2% |

All PCR constituents are mixed together and amplification occurs on a bench-top thermal cycler using the following protocol;

| **Temperature (°C)** | **Duration (seconds)** | **Cycles** |
|---|---|---|
| 98 | 60 | 1 |
| 98 | 1 | 35 |
| 60 | 2 | 35 |
| 72 | 2 | 35 |
| 72 | 60 | 1 |
| 4 | ∞ | - |

After PCR amplification has occurred, the PCR tube is spun down to separate amplified DNA and blood precipitate. At least a portion of the resulting amplified DNA material is applied to a detection region.

In this example, the detection region comprises a substrate onto which probes are printed. The detection region is prepared by printing the probes then washing to remove unbound probe and blocking to prevent unwanted non-specific binding to the substrate;

Washing;
- Wash slides to remove unbound probe molecules and buffer substances to avoid interference with subsequent hybridization experiments.
- Rinse 2 × 2 min in 0.2 % SDS at room temperature.
- Rinse 2 × 2 min in diH2O at room temperature.
- <optional>Denaturation step for arrays spotted with PCR-probes: 1 × 3 min in boiling diH2O.
- Rinse 1 × 1 min in diH2O at room temperature. **Note:** The volume of washing solution is at least 250 ml for 5 slides.
- Proceed to Blocking immediately.

Slides are not allowed to dry in between washing steps and between washing and blocking.

### Blocking;

Block the slides with Aldehyde Blocking Solution (made by dissolving 1.0 g NaBH₄ in 300 ml PBS and 100 ml ethanol) as follows:
- Incubate slides 1 × 15 min in Aldehyde Blocking Solution at room temperature. The volume of blocking solution should be at least 100 ml for 5 slides.
- Rinse 2 × 2 min in 0.2 % SDS at room temperature.
- Rinse 2 × 2 min in diH2O at room temperature.
- Dry the slides in an oil-free air or nitrogen stream or by centrifugation (200 × g for 5 min) to avoid any water stains on the slide surface.

The probe concentration is 100µM - 2 drops of volume 200picolitres per drop. Amino terminated probes are attached to the surface with a spacer.

The probes are organised in array to minimise any imaging bias. An exemplary array is shown in Figure 2A.

When using Cy5 labelled alignment probes, the fluorophore is positioned away from 3' end of probe.

### Hybridisation and Imaging

An adhesive hybridisation chamber with inlet and outlet ports is placed over the array of printed probes on the slide. The PCR product (amplicon mix/amplified DNA portion) is pipetted into the chamber to cover the array, and the array ports are sealed with adhesive seals.

The slide is hybridised for 1 hour at 50°C. During this time, hybridisation of the Cy5 labelled PCR strand to the capture probes will take place depending on which SNP is present in the amplicon.

Following the hybridisation, seals are removed and the unbound PCR product is aspirated out of the chambers.

To ensure all unbound nucleic acid amplicons is removed, 2x water washes are performed at 50°C followed by a room temperature wash in a 50ml falcon tube filled with water.

The slide is then dried using a slide centrifuge and imaged. Detection of the bound fluorescently labelled Cy5 amplicons is carried out using a fluorescence reader. An exemplary image is shown in Figure 2A (with the key to the probe printing being shown in Figure 2B) where the CYP2C9^{∗}2 Wildtype probes are visible whereas the mutant is not. The wildtype and mutant CYP2C9 ^{∗}3 probes are visible. The wildtype and mutant

VKORC1 probes are visible. The image of Figure 2A was analysed, giving the following results:

| | Median Fluorescence | Median - Neg Control | Discrimination Factor (Wildtype-neg/mutant-neg) | Genotype |
|---|---|---|---|---|
| Neg control | 15362.95 | | | |
| *^{∗}*2 Wild type | 37856.74 | 22493.79 | 52.22490771 | Wild Type Homozygous |
| *^{∗}*2 Mutant | 15793.66 | 430.71 | | |
| ^{∗}3 Wild type | 21372.19 | 6009.24 | 2.052251957 | Heterozygous |
| ^{∗}3 Mutant | 18291.07 | 2928.12 | | |
| VK Wild type | 52900.15 | 37537.2 | 2.895414814 | Heterozygous |
| VK Mutant | 28327.31 | 12964.36 | | |

It will be appreciated that features from one embodiment may be appropriately incorporated into another embodiment unless technically unfeasible to do so.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations).

It will be appreciated that various embodiments of the present disclosure have been described herein for purposes of illustration.

### References

Thomas Sebastian, Christopher G. Cooney, Jennifer Parker, Peter Qu, Alexander Perov, Julia B. Golova, Lindsay Pozza, Rafal M. Iwasiow, Rebecca Holmberg, Integrated amplification microarray system in a lateral flow cell for warfarin genotyping from saliva, Clinica Chimica Acta, Volume 429, 2014, Pages 198-205, ISSN 0009-8981, https://doi.org/10.1016/j.cca.2013.12.009.

Lyon Elaine, Gwen McMillin, Roberta Melis, Pharmacogenetic Testing for Warfarin Sensitivity, Clinics in Laboratory Medicine, Volume 28, Issue 4, 2008, Pages 525-537, ISSN 0272-2712, https://doi.org/10.1016/j.cll.2008.09.001.

Sheth Harsh Jayesh, Genetic determinants of response to aspirin and warfarin and development of silicon nanowire based genotyping, PhD Thesis, 2015, Newcastle University, Institute of Genetic Medicine, http://hdl.handle.net/10443/3042.

## Claims

1. A rapid method of determining warfarin sensitivity for use in a microfluidic device comprising; contacting Z a sample with pairs of oligonucleotide primers, each pair comprising a forward primer and a reverse primer, wherein the pairs of oligonucleotide primers comprise;
| **Primer name** | **Sequence** | **SEQ ID** |
|---|---|---|
| ^{∗}2_v1_F | GGCGTTTCTCCCTCATGACGC | SEQ ID 1 |
| ^{∗}2_v1_R | GAGTAGGGTCACCCACCCTTGG | SEQ ID 2 |
| ^{∗}3_v1_F | GCAAGACAGGAGCCACATGCC | SEQ ID 3 |
| ^{∗}3_v2_R or ^{∗}3_v2_R' | GAATTTAATGTCACAGGTCACTGC | SEQ ID 4 |
| | CTGAATTTAATGTCACAGGTCACTGCATG | SEQ ID 13 |
| VK_v1_F | GGATTATAGGCGTGAGCCACC | SEQ ID 5 |
| VK_v1_R | GGTAGGTGCAACAGTAAGGGATCC | SEQ ID 6 |
or wherein the pairs of oligonucleotide primers have at least 80% sequence homology to the primers identified as SEQ ID 1, 2, 3, 4, 5 and 6;
and are operable to bind genomic regions flanking *CYP2C9^{∗}2* (rs1799853), *CYP2C9^{∗}3 (rs1057910)* and *VKORC1*-*1639G>A* (rs9923231) single nucleotide polymorphism (SNP) sites and to amplify genomic regions to give amplicons comprising said SNP sites;
amplifying, substantially simultaneously, the SNP sites by polymerase chain reaction by annealing the primer pairs to the nucleic acid present in the sample; simultaneously extending the annealed primers to synthesize an extension product, wherein each extension product after separation from the nucleic acid, serves as a template for the synthesis of an extension product for the other primer of each pair; and
detecting the amplified products, if present, to indicate the presence or absence of a single nucleotide polymorphism (SNP) associated with warfarin sensitivity in the sample, wherein detection of single nucleotide polymorphisms is indicative of warfarin sensitivity.

2. A method as in Claim 1 wherein the pairs of oligonucleotide primers have at least 90% sequence homology, or at least, 95% sequence homology to the primers identified as SEQ ID 1, 2, 3, 4, 5 and 6.

3. A method as in any of the previous claims wherein the respective primer pair concentrations CYP2C9^{∗}2:CYP2C9^{∗}3:VKORC1 are 1:2:4 or 1:2:6.

4. A method as in any of the previous claims wherein for each primer pair, one of the pair is labelled.

5. A method as in any of the previous Claims wherein the detecting includes the step of providing a plurality of capture probes capable of selectively binding to either wild type or mutant versions Z CYP2C9*2(rs1799853), *CYP2C9^{∗}3(rs1057910)* and *VKORC1-*1639G>A, wherein the binding of amplified products to capture probes indicates the presence or absence of a SNP associated with warfarin sensitivity in the clinical sample, wherein the capture probes comprise;
| **Probe name** | **Sequence** | **SEQ ID** |
|---|---|---|
| ^{∗}2-WT probe | GAA CAC GGT CCT CAA TGC T | SEQ ID No 7 |
| ^{∗}2-M probe | GAA CAC AGT CCT CAA TGC T | SEQ ID No 8 |
| ^{∗}3-WT probe | ACG AGG TCC AGA GAT ACA TTG AC | SEQ ID No 9 |
| ^{∗}3-M probe | CGA GGT CCA GAG ATA CCT TGA C | SEQ ID No 10 |
| VK-WT probe | CAA CCA TTG GCC GGG T | SEQ ID No 11 |
| VK-M probe | CAA CCA TTG GCC AGG T | SEQ ID No 12 |
and are the probes identified as SEQ ID No's 7, 8 9, 10, 11 and 12 or have at least 80% sequence homology, or at least 90% sequence homology, or at least 95% sequence homology to the probes identified as SEQ ID No's 7, 8 9, 10, 11 and 12.

6. A kit for rapid determination of warfarin sensitivity and for use in a microfluidic device, comprising pairs of oligonucleotide primers, each pair comprising a forward primer and a reverse primer, wherein the pairs of oligonucleotide primers comprise;
| **Primer name** | **Sequence** | **SEQ ID** |
|---|---|---|
| ^{∗}2_v1_F | GGCGTTTCTCCCTCATGACGC | SEQ ID 1 |
| ^{∗}2_v1_R | GAGTAGGGTCACCCACCCTTGG | SEQ ID 2 |
| ^{∗}3_v1_F | GCAAGACAGGAGCCACATGCC | SEQ ID 3 |
| ^{∗}3_v2_R or ^{∗}3_v2_R' | GAATTTAATGTCACAGGTCACTGC | SEQ ID 4 |
| | CTGAATTTAATGTCACAGGTCACTGCATG | SEQ ID 13 |
| VK_v1_F | GGATTATAGGCGTGAGCCACC | SEQ ID 5 |
| VK_v1_R | GGTAGGTGCAACAGTAAGGGATCC | SEQ ID 6 |
or wherein the pairs of oligonucleotide primers have at least 80% sequence homology to the primers identified in SEQ ID No's 1, 2, 3, 4, 5;
and are operable to bind genomic regions flanking CYP2C9*2(rs1799853) Z
*CYP2C9^{∗}3(rs1057910)* and *VKORC1*-*1639G>A* single nucleotide polymorphism (SNP) sites and to amplify genomic regions to give amplicons comprising said SNP sites; and reagents for carrying out a polymerase chain reaction, including a polymerase.

7. A kit as in Claim 6 wherein the primers are the primers as identified in SEQ ID No's 1, 2, 3, 4, 5 and 6.

8. A kit as in Claim 6 wherein the primers have at least 90% sequence homology, or at least 95% sequence homology, to the primers identified in SEQ ID No's 1, 2, 3, 4, 5 and 6.

9. A kit as in any of Claims 6 to 8, further including a plurality of capture probes capable of selectively binding to either wild type or mutant versions of CYP2C9*2(rs1799853), *CYP2C9^{∗}3(rs1057910)* and *VKORC1*-*1639G>A.*

10. A kit as in Claim 9 wherein the probes comprise;
| **Probe name** | **Sequence** | **SEQ ID** |
|---|---|---|
| ^{∗}2-WT probe | GAA CAC GGT CCT CAA TGC T | SEQ ID No 7 |
| ^{∗}2-M probe | GAA CAC AGT CCT CAA TGC T | SEQ ID No 8 |
| ^{∗}3-WT probe | ACG AGG TCC AGA GAT ACA TTG AC | SEQ ID No 9 |
| ^{∗}3-M probe | CGA GGT CCA GAG ATA CCT TGA C | SEQ ID No 10 |
| VK-WT probe | CAA CCA TTG GCC GGG T | SEQ ID No 11 |
| VK-M probe | CAA CCA TTG GCC AGG T | SEQ ID No 12 |

11. A kit as in any of Claims 9 or 10 wherein the capture probes are the probes identified as SEQ ID No's 7, 8, 9, 10, 11 and 12 or have at least 80% sequence homology, or at least 90% sequence homology, or at least, 95% sequence homology to the capture probes identified as SEQ ID No's 7, 8 9, 10, 11 and 12.

12. A kit as in any of Claims 9 to 11 wherein the capture probes are bound to sensitive detection devices that may be nanowires.

13. A microfluidic device for rapid determination of warfarin sensitivity, comprising;
a sample reception port;
an amplification region for amplifying genomic regions to give amplicons comprising CYP2C9*2(rs1799853), *CYP2C9^{∗}3(rs1057910)* and *VKORC1-1639G>A* single nucleotide polymorphism (SNP) sites, said region in use containing pairs of oligonucleotide primers, each pair comprising a forward primer and a reverse primer, wherein the pairs of oligonucleotide primers comprise;
| **Primer name** | **Sequence** | **SEQ ID** |
|---|---|---|
| ^{∗}2_v1_F | GGCGTTTCTCCCTCATGACGC | SEQ ID 1 |
| ^{∗}2_v1_R | GAGTAGGGTCACCCACCCTTGG | SEQ ID 2 |
| ^{∗}3_v1_F | GCAAGACAGGAGCCACATGCC | SEQ ID 3 |
| ^{∗}3_v2_R or ^{∗}3_v2_R' | GAATTTAATGTCACAGGTCACTGC | SEQ ID 4 |
| | CTGAATTTAATGTCACAGGTCACTGCATG | SEQ ID 13 |
| VK_v1_F | GGATTATAGGCGTGAGCCACC | SEQ ID 5 |
| VK_v1_R | GGTAGGTGCAACAGTAAGGGATCC | SEQ ID 6 |
or wherein the pairs of oligonucleotide primers have at least 80% sequence homology to the primers identified in SEQ ID No's 1, 2, 3, 4, 5 and 6;
and are operable to bind genomic regions flanking CYP2C9*2(rs1799853)
*CYP2C9^{∗}3(rs1057910)* and *VKORC1*-*1639G>A* single nucleotide polymorphism (SNP) sites;
a detection region, said detection region being downstream of the amplification region, for detecting the amplicons to indicate the presence or absence of a single nucleotide polymorphism (SNP) associated with warfarin sensitivity in the sample.

14. A device as in Claim 13 wherein the primers have at least 90% sequence homology, or at least 95% sequence homology, to the primers identified in SEQ ID No's 1, 2, 3, 4, 5 and 6.

15. A device as in any of Claims 13 or 14 wherein the detection region comprises a plurality of capture probes comprising;
| **Probe name** | **Sequence** | **SEQ ID** |
|---|---|---|
| ^{∗}2-WT probe | GAA CAC GGT CCT CAA TGC T | SEQ ID No 7 |
| ^{∗}2-M probe | GAA CAC AGT CCT CAA TGC T | SEQ ID No 8 |
| ^{∗}3-WT probe | ACG AGG TCC AGA GAT ACA TTG AC | SEQ ID No 9 |
| ^{∗}3-M probe | CGA GGT CCA GAG ATA CCT TGA C | SEQ ID No 10 |
| VK-WT probe | CAA CCA TTG GCC GGG T | SEQ ID No 11 |
| VK-M probe | CAA CCA TTG GCC AGG T | SEQ ID No 12 |
that are capable of selectively binding to either wild type or mutant versions of CYP2C9*2(rs1799853), CYP2C9^{∗}3(rs1057910) and *VKORC1-1639G>A* and wherein the capture probes are the probes identified as SEQ ID No's 7, 8, 9, 10, 11 and 12 or have at least 80% sequence homology, or at least 90% sequence homology, or at least, 95% sequence homology to the capture probes identified as SEQ ID No's 7, 8 9, 10, 11 and 12.

## Patentansprüche

1. Schnellverfahren zum Bestimmen von Warfarin-Sensitivität zur Verwendung in einer mikrofluidischen Vorrichtung, umfassend;
Inkontaktbringen einer Probe mit Paaren von Oligonukleotidprimern, wobei jedes Paar einen Vorwärtsprimer und einen Rückwärtsprimer umfasst, wobei die Paare von Oligonukleotidprimern umfassen;
| Primername | Sequenz | SEQ ID |
|---|---|---|
| ^{∗}2_v1_F | GGCGTTTCTCCCTCATGACGC | SEQ 1 |
| ^{∗}2_vl_R | GAGTAGGGTCACCCACCCTTGG | SEQ 2 |
| ^{∗}3_v1_F | GCAAGACAGGAGCCACATGCC | SEQ ID 3 |
| ^{∗}3_v2_R oder ^{∗}3_v2_R' | GAATTTAATGTCACAGGTCACTGC | SEQ ID 4 |
| | CTGAATTTAATGTCACAGGTCACTGCATG | SEQID 13 |
| VK_vl_F | GGATTATAGGCGTGAGCCACC | SEQ ID 5 |
| VK_vl_R | GGTAGGTGCAACAGTAAGGGATCC | SEQ ID 6 |
oder wobei die Paare von Oligonukleotidprimern mindestens 80 % Sequenzhomologie zu den als SEQ ID 1, 2, 3, 4, 5 und 6 identifizierten Primern aufweisen;
und funktionsfähig sind, um genomische Regionen zu binden, die *CYP2C9^{∗}2* (rs1799853), *CYP2C9^{∗}3 (rs1057910)* und *VKORC1-1639G>A* (rs9923231) - Einzelnukleotidpolymorphismus-(SNP)-Stellen flankieren, und genomische Regionen zu amplifizieren, um Amplikons zu ergeben, die die SNP-Stellen umfassen;
im Wesentlichen gleichzeitiges Amplifizieren der SNP-Stellen durch PolymeraseKettenreaktion durch Anlagern der Primerpaare an die in der Probe vorhandene Nukleinsäure; gleichzeitiges Verlängern der angelagerten Primer, um ein Verlängerungsprodukt zu synthetisieren, wobei jedes Verlängerungsprodukt nach der Trennung von der Nukleinsäure als Templat für die Synthese eines Verlängerungsprodukts für den anderen Primer jedes Paares dient; und
Nachweisen der amplifizierten Produkte, falls vorhanden, um das Vorhandensein oder Fehlen eines mit Warfarin-Sensitivität assoziierten Einzelnukleotidpolymorphismus (SNP) in der Probe anzuzeigen, wobei der Nachweis von Einzelnukleotidpolymorphismen eine Warfarin-Sensitivität anzeigt.

2. Verfahren nach Anspruch 1, wobei die Paare von Oligonukleotidprimern mindestens 90 % Sequenzhomologie oder mindestens 95 % Sequenzhomologie zu den als SEQ ID 1, 2, 3, 4, 5 und 6 identifizierten Primern aufweisen.

3. Verfahren nach einem der vorstehenden Ansprüche, wobei die jeweiligen Primerpaarkonzentrationen CYP2C9^{∗}2:CYP2C9^{∗}3:VKORC1 1:2:4 oder 1:2:6 betragen.

4. Verfahren nach einem der vorstehenden Ansprüche, wobei für jedes Primerpaar einer des Paares markiert wird.

5. Verfahren nach einem der vorstehenden Ansprüche, wobei das Nachweisen den Schritt des Bereitstellens einer Vielzahl von Einfangsonden einschließt, die selektiv entweder an Wildtyp- oder mutierte Versionen CYP2C9*2(rs1799853), *CYP2C9^{∗}3(rs1057910)* und *VKORC1-1639G>A* binden können, wobei die Bindung von amplifizierten Produkten an Einfangsonden das Vorhandensein oder Fehlen eines mit Warfarin-Sensitivität assoziierten SNP in der klinischen Probe anzeigt, wobei die Einfangsonden umfassen;
| Sondenname | Sequenz | SEQ ID |
|---|---|---|
| ^{∗}2-WT-Sonde | GAA CAC GGT CCT CAA TGC T | SEQ ID Nr. 7 |
| ^{∗}2-M-Sonde | GAA CAC AGT CCT CAA TGC T | SEQ ID Nr. 8 |
| ^{∗}3-WT-Sonde | ACG AGG TCC AGA GAT ACA TTG AC | SEQ ID Nr. 9 |
| ^{∗}3-M-Sonde | CGA GGT CCA GAG ATA CCT TGA C | SEQ ID Nr. 10 |
| VK-WT-Sonde | CAA CCA TTG GCC GGG T | SEQ ID Nr. 11 |
| VK-M-Sonde | CAA CCA TTG GCC AGG T | SEQ ID Nr. 12 |
und die als SEQ ID Nr. 7, 8 9, 10, 11 und 12 identifizierten Sonden sind oder mindestens 80 % Sequenzhomologie oder mindestens 90 % Sequenzhomologie oder mindestens 95 % Sequenzhomologie zu den als SEQ ID identifizierten Sonden Nr. 7, 8, 9, 10, 11 und 12 aufweisen.

6. Kit zur schnellen Bestimmung von Warfarin-Sensitivität und zur Verwendung in einer mikrofluidischen Vorrichtung, umfassend Paare von Oligonukleotidprimern, wobei jedes Paar einen Vorwärtsprimer und einen Rückwärtsprimer umfasst, wobei die Paare von Oligonukleotidprimern umfassen;
| Primername | Sequenz | SEQ ID |
|---|---|---|
| ^{∗}2_vl_F | GGCGTTTCTCCCTCATGACGC | SEQ ID 1 |
| ^{∗}2_vl_R | GAGTAGGGTCACCCACCCTTGG | SEQ ID 2 |
| ^{∗}3_v1_F | GCAAGACAGGAGCCACATGCC | SEQ ID 3 |
| ^{∗}3_v2_R | GAATTTAATGTCACAGGTCACTGC | SEQ ID 4 |
| oder ^{∗}3_v2_R' | CTGAATTTAATGTCACAGGTCACTGCATG | SEQID 13 |
| VK_vl_F | GGATTATAGGCGTGAGCCACC | SEQ ID 5 |
| VK_vl_R | GGTAGGTGCAACAGTAAGGGATCC | SEQ ID 6 |
oder wobei die Paare von Oligonukleotidprimern mindestens 80 % Sequenzhomologie zu den in SEQ ID Nr. 1, 2, 3, 4, 5 identifizierten Primern aufweisen;
und funktionsfähig sind, um genomische Regionen zu binden, die CYP2C9^{∗}2 (rs1799853), *CYP2C9^{∗}3 (rs1057910)* und *VKORC1-1639G>A* - Einzelnukleotidpolymorphismus-(SNP)-Stellen flankieren, und genomische Regionen zu amplifizieren, um Amplikons zu ergeben, die die SNP-Stellen umfassen; und
Reagenzien zum Durchführen einer Polymerasekettenreaktion, einschließlich einer Polymerase.

7. Kit nach Anspruch 6, wobei die Primer die in SEQ ID Nr. 1, 2, 3, 4, 5 und 6 identifizierten Primer sind.

8. Kit nach Anspruch 6, wobei die Primer mindestens 90 % Sequenzhomologie oder mindestens 95 % Sequenzhomologie zu den in SEQ ID Nr. 1, 2, 3, 4, 5 und 6 identifizierten Primern aufweisen.

9. Kit nach einem der Ansprüche 6 bis 8, weiter umfassend eine Vielzahl von Einfangsonden, die in der Lage sind, selektiv entweder an Wildtyp- oder mutierte Versionen von CYP2C9*2(rs1799853), *CYP2C9^{∗}3(rs1057910)* und *VKORC1-1639G>A* zu binden.

10. Kit nach Anspruch 9, wobei die Sonden umfassen;
| Sondenname | Sequenz | SEQ ID |
|---|---|---|
| ^{∗}2-WT-Sonde | GAA CAC GGT CCT CAA TGC T | SEQ ID Nr. 7 |
| ^{∗}2-M-Sonde | GAA CAC AGT CCT CAA TGC T | SEQ ID Nr. 8 |
| ^{∗}3-WT-Sonde | ACG AGG TCC AGA GAT ACA TTG AC | SEQ ID Nr. 9 |
| ^{∗}3-M-Sonde | CGA GGT CCA GAG ATA CCT TGA C | SEQ ID Nr. 10 |
| VK-WT-Sonde | CAA CCA TTG GCC GGG T | SEQ ID Nr. 11 |
| VK-M-Sonde | CAA CCA TTG GCC AGG T | SEQ ID Nr. 12 |

11. Kit nach einem der Ansprüche 9 oder 10, wobei die Erfassungssonden die als SEQ ID Nr. 7, 8, 9, 10, 11 und 12 identifizierten Sonden sind oder mindestens 80 % Sequenzhomologie oder mindestens 90 % Sequenzhomologie oder mindestens 95 % Sequenzhomologie zu den als SEQ ID Nr. 7, 8, 9, 10, 11 und 12 identifizierten Einfangsonden aufweisen.

12. Kit nach einem der Ansprüche 9 bis 11, wobei die Einfangsonden an empfindliche Nachweisvorrichtungen gebunden sind, die Nanodrähte sein können.

13. Mikrofluidische Vorrichtung zur schnellen Bestimmung von Warfarin-Sensitivität, umfassend;
eine Probenaufnahmeöffnung;
eine Amplifikationsregion zum Amplifizieren genomischer Regionen, um Amplikons zu ergeben, die CYP2C9*2(rs1799853), *CYP2C9^{∗}3(rs1057910)* und *VKORC1-1639G>A-*Einzelnukleotidpolymorphismus-(SNP)-Stellen umfassen, wobei die verwendete Region Paare von Oligonukleotidprimern enthält, wobei jedes Paar einen Vorwärtsprimer und einen Rückwärtsprimer umfasst, wobei die Paare von Oligonukleotidprimern umfassen;
| Primername | Sequenz | SEQ ID |
|---|---|---|
| ^{∗}2_vI_F | GGCGTTTCTCCCTCATGACGC | SEQ ID 1 |
| ^{∗}2_vI_R | GAGTAGGGTCACCCACCCTTGG | SEQ ID 2 |
| ^{∗}3_vI_F | GCAAGACAGGAGCCACATGCC | SEQ ID 3 |
| ^{∗}3_v2_R oder ^{∗}3_v2_R' | GAATTTAATGTCACAGGTCACTGC CTGAATTTAATGTCACAGGTCACTGCATG | SEQ ID 4 SEQID 13 |
| VK_vI_F | GGATTATAGGCGTGAGCCACC | SEQ ID 5 |
| VK_vl_R | GGTAGGTGCAACAGTAAGGGATCC | SEQ ID 6 |
oder wobei die Paare von Oligonukleotidprimern mindestens 80 % Sequenzhomologie zu den in SEQ ID Nr. 1, 2, 3, 4, 5 und 6 identifizierten Primern aufweisen;
und funktionsfähig sind, um genomische Regionen zu binden, die CYP2C9*2(rs1799853), *CYP2C9^{∗}3(rs1057910)* und *VKORC1*-*1639G>A-*Einzelnukleotidpolymorphismus-(SNP)-Stellen flankieren;
eine Nachweisregion, wobei die Nachweisregion stromabwärts von der Amplifikationsregion liegt, zum Nachweisen der Amplikons, um das Vorhandensein oder Fehlen eines mit Warfarin-Sensitivität assoziierten Einzelnukleotidpolymorphismus (SNP) in der Probe anzuzeigen.

14. Vorrichtung nach Anspruch 13, wobei die Primer mindestens 90 % Sequenzhomologie oder mindestens 95 % Sequenzhomologie zu den in SEQ . ID Nr. 1, 2, 3, 4, 5 und 6 identifizierten Primern aufweisen.

15. Vorrichtung nach einem der Ansprüche 13 oder 14, wobei die Nachweisregion eine Vielzahl von Einfangsonden umfasst, umfassend;
| Sondenname | Sequenz | SEQ ID |
|---|---|---|
| ^{∗}2-WT-Sonde | GAA CAC GGT CCT CAA TGC T | SEQ ID Nr. 7 |
| ^{∗}2-M-Sonde | GAA CAC AGT CCT CAA TGC T | SEQ ID Nr. 8 |
| ^{∗}3-WT-Sonde | ACG AGG TCC AGA GAT ACA TTG AC | SEQ ID Nr. 9 |
| ^{∗}3-M-Sonde | CGA GGT CCA GAG ATA CCT TGA C | SEQ ID Nr. 10 |
| VK-WT-Sonde | CAA CCA TTG GCC GGG T | SEQ ID Nr. 11 |
| VK-M-Sonde | CAA CCA TTG GCC AGG T | SEQ ID Nr. 12 |
die in der Lage sind, selektiv an entweder Wildtyp- oder mutierte Versionen von CYP2C9*2(rs1799853), *CYP2C9^{∗}3(rs1057910)* und VKORC1-1639G>A zu binden, und wobei die Erfassungssonden die als SEQ ID Nr. 7, 8, 9, 10, 11 und 12 identifizierten Sonden sind oder mindestens 80 % Sequenzhomologie oder mindestens 90 % Sequenzhomologie oder mindestens 95 % Sequenzhomologie zu den als SEQ ID Nr. 7, 8, 9, 10, 11 und 12 identifizierten Einfangsonden aufweisen.

## Revendications

1. Procédé rapide de détermination de la sensibilité à la warfarine destiné à être utilisé dans un dispositif microfluidique comprenant ;
la mise en contact d'un échantillon avec des paires d'amorces oligonucléotidiques, chaque paire comprenant une amorce directe et une amorce inverse, dans lequel les paires d'amorces oligonucléotidiques comprennent ;
| **Nom de l'amorce** | **Séquence** | **SEQ ID** |
|---|---|---|
| ^{∗}2_v1_F | GGCGTTTCTCCCTCATGACGC | SEQ ID 1 |
| ^{∗}2_v1_R | GAGTAGGGTCACCCACCCTTGG | SEQ ID 2 |
| ^{∗}3_v1_F | GCAAGACAGGAGCCACATGCC | SEQ ID 3 |
| ^{∗}3_v2_R ou ^{∗}3_v2_R' | GAATTTAATGTCACAGGTCACTGC CTGAATTTAATGTCACAGGTCACTGCATG | SEQ ID 4 SEQ ID 13 |
| VK_v1_F | GGATTATAGGCGTGAGCCACC | SEQ ID 5 |
| VK_v1_R | GGTAGGTGCAACAGTAAGGGATCC | SEQ ID 6 |
ou dans lequel les paires d'amorces oligonucléotidiques présentent au moins 80 % d'homologie de séquence avec les amorces identifiées comme SEQ ID 1, 2, 3, 4, 5 et 6 ;
et sont utilisables pour lier des régions génomiques flanquant les sites de polymorphisme mononucléotidique (SNP) de *CYP2C9^{∗}2* (rs1799853), *CYP2C9^{∗}3* (rs1057910) et *VKORC1*-1639G>A (rs9923231) et pour amplifier les régions génomiques pour donner des amplicons comprenant lesdits sites SNP ;
l'amplification, sensiblement simultanément, des sites SNP par réaction en chaîne par polymérase en hybridant les paires d'amorces à l'acide nucléique présent dans l'échantillon ; en étendant simultanément les amorces hybridées pour synthétiser un produit d'extension, dans lequel chaque produit d'extension, après séparation de l'acide nucléique, sert de matrice pour la synthèse d'un produit d'extension pour l'autre amorce de chaque paire ; et
la détection des produits amplifiés, si présents, pour indiquer la présence ou l'absence d'un polymorphisme mononucléotidique (SNP) associé à la sensibilité à la warfarine dans l'échantillon, dans lequel la détection de polymorphismes mononucléotidiques indique la sensibilité à la warfarine.

2. Procédé selon la revendication 1, dans lequel les paires d'amorces oligonucléotidiques présentent au moins 90 % d'homologie de séquence, ou au moins 95 % d'homologie de séquence avec les amorces identifiées comme SEQ ID 1, 2, 3, 4, 5 et 6.

3. Procédé selon l'une quelconque des revendications précédentes, dans lequel les concentrations de paire d'amorces respectives CYP2C9^{∗}2:CYP2C9^{∗}3:VKORC1 sont de 1:2:4 ou 1:2:6.

4. Procédé selon l'une quelconque des revendications précédentes, dans lequel pour chaque paire d'amorces, une de la paire est marquée.

5. Procédé selon l'une quelconque des revendications précédentes, dans lequel la détection inclut l'étape consistant à fournir une pluralité de sondes de capture capables de se lier sélectivement aux versions de type sauvage ou mutantes CYP2C9*2(rs1799853), *CYP2C9^{∗}3(rs1057910)* et *VKORC1*-*1639G>A*, dans lequel la liaison de produits amplifiés aux sondes de capture indique la présence ou l'absence d'un SNP associé à la sensibilité à la warfarine dans l'échantillon clinique, dans lequel les sondes de capture comprennent ;
| **Nom de la sonde** | **Séquence** | **SEQ ID** |
|---|---|---|
| Sonde ^{∗} 2-WT | GAA CAC GGT CCT CAA TGC T | SEQ ID No 7 |
| Sonde ^{∗} 2-M | GAA CAC AGT CCT CAA TGC T | SEQ ID No 8 |
| Sonde ^{∗} 3-WT | ACG AGG TCC AGA GAT ACA TTG AC | SEQ ID No 9 |
| Sonde ^{∗} 3-M | CGA GGT CCA GAG ATA CCT TGA C | SEQ ID No 10 |
| Sonde VK-WT | CAA CCA TTG GCC GGG T | SEQ ID No 11 |
| Sonde VK-M | CAA CCA TTG GCC AG G T | SEQ ID No 12 |
et sont les sondes identifiées comme SEQ ID No 7, 8, 9, 10, 11 et 12 ou présentent au moins 80 % d'homologie de séquence, ou au moins 90 % d'homologie de séquence, ou au moins 95 % d'homologie de séquence avec les sondes identifiées comme SEQ ID No 7, 8, 9, 10, 11 et 12.

6. Kit pour la détermination rapide de la sensibilité à la warfarine et destiné à être utilisé dans un dispositif microfluidique, comprenant des paires d'amorces oligonucléotidiques, chaque paire comprenant une amorce directe et une amorce inverse, dans lequel les paires d'amorces oligonucléotidiques comprennent ;
| **Nom de l'amorce** | **Séquence** | **SEQ ID** |
|---|---|---|
| ^{∗}2_v1_F | GGCGTTTCTCCCTCATGACGC | SEQ ID 1 |
| ^{∗}2_v1_R | GAGTAGGGTCACCCACCCTTGG | SEQ ID 2 |
| ^{∗}3_v1_F | GCAAGACAGGAGCCACATGCC | SEQ ID 3 |
| ^{∗}3_v2_R ou ^{∗}3_v2_R' | GAATTTAATGTCACAGGTCACTGC CTGAATTTAATGTCACAGGTCACTGCATG | SEQ ID 4 SEQ ID 13 |
| VK_v1_F | GGATTATAGGCGTGAGCCACC | SEQ ID 5 |
| VK_v1_R | GGTAGGTGCAACAGTAAGGGATCC | SEQ ID 6 |
ou dans lequel les paires d'amorces oligonucléotidiques présentent au moins 80 % d'homologie de séquence avec les amorces identifiées dans SEQ ID No 1, 2, 3, 4, 5 ;
et sont utilisables pour lier les régions génomiques flanquant les sites de polymorphisme mononucléotidique (SNP) de CYP2C9*2(rs1799853), *CYP2C9^{∗}3(rs1057910)* et *VKORC1*-*1639G>A* et pour amplifier les régions génomiques pour donner des amplicons comprenant lesdits sites SNP ; et des réactifs pour effectuer une réaction en chaîne par polymérase, incluant une polymérase.

7. Kit selon la revendication 6, dans lequel les amorces sont les amorces telles qu'identifiées dans SEQ ID No 1, 2, 3, 4, 5 et 6.

8. Kit selon la revendication 6, dans lequel les amorces présentent au moins 90 % d'homologie de séquence, ou au moins 95 % d'homologie de séquence avec les amorces identifiées dans SEQ ID No 1, 2, 3, 4, 5 et 6.

9. Kit selon l'une quelconque des revendications 6 à 8, incluant en outre une pluralité de sondes de capture capables de se lier sélectivement aux versions de type sauvage ou mutantes de CYP2C9*2(rs1799853), *CYP2C9^{∗}3(rs1057910)* et *VKORC1-1639G>A.*

10. Kit selon la revendication 9, dans lequel les sondes comprennent ;
| **Nom de la sonde** | **Séquence** | **SEQ ID** |
|---|---|---|
| Sonde ^{∗} 2-WT | GAA CAC GGT CCT CAA TGC T | SEQ ID No 7 |
| Sonde ^{∗} 2-M | GAA CAC AGT CCT CAA TGC T | SEQ ID No 8 |
| Sonde ^{∗} 3-WT | ACG AGG TCC AGA GAT ACA TTG AC | SEQ ID No 9 |
| Sonde ^{∗} 3-M | CGA GGT CCA GAG ATA CCT TGA C | SEQ ID No 10 |
| Sonde VK-WT | CAA CCA TTG GCC GGG T | SEQ ID No 11 |
| Sonde VK-M | CAA CCA TTG GCC AG G T | SEQ ID No 12 |

11. Kit selon l'une quelconque des revendications 9 ou 10, dans lequel les sondes de capture sont les sondes identifiées comme SEQ ID No 7, 8, 9, 10, 11 et 12 ou présentent au moins 80 % d'homologie de séquence, ou au moins 90 % d'homologie de séquence, ou au moins 95 % d'homologie de séquence avec les sondes de capture identifiées comme SEQ ID No 7, 8, 9, 10, 11 et 12.

12. Kit selon l'une quelconque des revendications 9 à 11, dans lequel les sondes de capture sont liées à des dispositifs de détection sensibles qui peuvent être des nanofils.

13. Dispositif microfluidique pour la détermination rapide de la sensibilité à la warfarine, comprenant ; un port de réception d'échantillon ;
une région d'amplification pour amplifier des régions génomiques pour donner des amplicons comprenant des sites de polymorphisme mononucléotidique (SNP) CYP2C9*2(rs1799853), *CYP2C9^{∗}3(rs1057910)* et *VKORC1-1639G>A,* ladite région utilisée contenant des paires d'amorces oligonucléotidiques, chaque paire comprenant une amorce directe et une amorce inverse, dans lequel les paires d'amorces oligonucléotidiques comprennent ;
| **Nom de l'amorce** | **Séquence** | **SEQ ID** |
|---|---|---|
| ^{∗}2_v1_F | GGCGTTTCTCCCTCATGACGC | SEQ ID 1 |
| ^{∗}2_v1_R | GAGTAGGGTCACCCACCCTTGG | SEQ ID 2 |
| ^{∗}3_v1_F | GCAAGACAGGAGCCACATGCC | SEQ ID 3 |
| ^{∗}3_v2_R ou ^{∗}3_v2_R' | GAATTTAATGTCACAGGTCACTGC CTGAATTTAATGTCACAGGTCACTGCATG | SEQ ID 4 SEQ ID 13 |
| VK_v1_F | GGATTATAGGCGTGAGCCACC | SEQ ID 5 |
| VK_v1_R | GGTAGGTGCAACAGTAAGGGATCC | SEQ ID 6 |
ou dans lequel les paires d'amorces oligonucléotidiques présentent au moins 80 % d'homologie de séquence avec les amorces identifiées dans SEQ ID No 1, 2, 3, 4, 5 et 6 ;
et sont utilisables pour lier les régions génomiques flanquant les sites de polymorphisme mononucléotidique (SNP) de CYP2C9*2(rs1799853), *CYP2C9^{∗}3(rs1057910)* et *VKORC1-1639G>A ;*
une région de détection, ladite région de détection étant en aval de la région d'amplification, pour détecter les amplicons pour indiquer la présence ou l'absence d'un polymorphisme mononucléotidique (SNP) associé à la sensibilité à la warfarine dans l'échantillon.

14. Dispositif selon la revendication 13, dans lequel les amorces présentent au moins 90 % d'homologie de séquence, ou au moins 95 % d'homologie de séquence avec les amorces identifiées dans SEQ ID No 1, 2, 3, 4, 5 et 6.

15. Dispositif selon l'une quelconque des revendications 13 ou 14, dans lequel la région de détection comprend une pluralité de sondes de capture comprenant :
| **Nom de la sonde** | **Séquence** | **SEQ ID** |
|---|---|---|
| Sonde ^{∗} 2-WT | GAA CAC GGT CCT CAA TGC T | SEQ ID No 7 |
| Sonde ^{∗} 2-M | GAA CAC AGT CCT CAA TGC T | SEQ ID No 8 |
| Sonde ^{∗} 3-WT | ACG AGG TCC AGA GAT ACA TTG AC | SEQ ID No 9 |
| Sonde ^{∗} 3-M | CGA GGT CCA GAG ATA CCT TGA C | SEQ ID No 10 |
| Sonde VK-WT | CAA CCA TTG GCC GGG T | SEQ ID No 11 |
| Sonde VK-M | CAA CCA TTG GCC AG G T | SEQ ID No 12 |
qui sont capables de se lier sélectivement aux versions de type sauvage ou mutantes de CYP2C9*2(rs1799853), CYP2C9^{∗}3(rs1057910) et VKORC1-1639G>A et dans lequel dans lequel les sondes de capture sont les sondes identifiées comme SEQ ID No 7, 8, 9,10, 11 et 12 ou présentent au moins 80 % d'homologie de séquence, ou au moins 90 % d'homologie de séquence, ou au moins 95 % d'homologie de séquence avec les sondes de capture identifiées comme SEQ ID No 7, 8, 9, 10, 11 et 12.
